(19)

**Europäisches Patentamt**
**European Patent Office**
**Office européen des brevets**

(11) **EP 4 585 679 A1**

(12) **EUROPEAN PATENT APPLICATION**
published in accordance with Art. 153(4) EPC

(43) Date of publication:
**16.07.2025 Bulletin 2025/29**

(21) Application number: **23862945.5**

(22) Date of filing: **24.08.2023**

(51) International Patent Classification (IPC):
***C12N 5/071*** (2010.01)    ***C12N 1/00*** (2006.01)

(52) Cooperative Patent Classification (CPC):
**C12N 1/00; C12N 5/06**

(86) International application number:
**PCT/JP2023/030444**

(87) International publication number:
**WO 2024/053406 (14.03.2024 Gazette 2024/11)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **09.09.2022 JP 2022143939**

(71) Applicants:
• **OSAKA UNIVERSITY**
  **Suita-shi**
  **Osaka 565-0871 (JP)**

• **National Institutes of Biomedical Innovation,
  Health and Nutrition**
  **Ibaraki-shi, Osaka 567-0085 (JP)**

(72) Inventors:
• **MIZUGUCHI, Hiroyuki**
  **Suita-shi, Osaka 565-0871 (JP)**
• **KAWAI, Kanae**
  **Suita-shi, Osaka 565-0871 (JP)**
• **INUI, Tatsuya**
  **Suita-shi, Osaka 565-0871 (JP)**

(74) Representative: **Hertin und Partner
Rechts- und Patentanwälte PartG mbB
Kurfürstendamm 63
10707 Berlin (DE)**

(54) **SMALL INTESTINE EPITHELIUM-LIKE CELLS AND PRODUCTION METHOD THEREOF**

(57)    While studies have been made on a method of selectively inducing differentiation from pluripotent stem cells into enterocyte-like cells, provided is an excellent and high-functionality enterocyte-like cell population that can be stably tested for multi-drug metabolism and permeability, and also provided an efficient method of producing cells. The method of inducing differentiation from pluripotent stem cells into enterocyte-like cells includes the following steps (1) and (2): (1) a step of inducing differentiation from pluripotent stem cells into definitive endoderm cells; and (2) a step of culturing the definitive endoderm cells in a system containing CHIR99021, followed by induction of differentiation into intestinal progenitor cells. A cell population of enterocyte-like cells is more effectively obtained by seeding and culturing enterocyte-like cells produced by the method of inducing differentiation, in a base material for organoid production or a base material for two-dimensional culture.

FIG. 2

EP 4 585 679 A1

**Description**

Technical Field

The present invention relates to a method of inducing differentiation from pluripotent stem cells into enterocyte-like cells (ELCs), and to enterocyte-like cells obtained by the method of inducing differentiation.

The present application claims priority from Japanese Patent Application No. 2022-143939, which is incorporated herein by reference.

Background Art

A drug administered orally is first absorbed, metabolized, and excreted in the small intestine. In pharmacokinetics in the absorption, the metabolism, the excretion, and the like, influx transporters, such as solute carrier family 15 member 1 (SLC15A1)/peptide transporter 1 (PEPT1); drug metabolic enzymes such as cytochrome P450 family 3 subfamily A member 4 (CYP3A4), and efflux transporters, such as P-glycoprotein (P-gp)/ATP binding cassette subfamily B member 1 (ABCB1)/multiple drug resistance 1 (MDR1) and ATP binding cassette subfamily G member 2 (ABCG2)/breast cancer resistance protein (BCRP), have major roles. In vitro evaluation of pharmacokinetics is important to effectively develop a safe pharmaceutical product.

Human primary enterocytes are not easily available, and have problems of differences in properties due to individual variability. Further, it is difficult to culture the obtained cells over a long period while maintaining their functions. Currently, in vitro intestinal pharmacokinetic studies generally have employed intestinal inversion with use of an intestinal tissue derived from a small animal such as a rat, a test with use of an artificial lipid membrane, an evaluation system with use of a cell line such as Caco-2 cells (a cell line derived from human colon cancer), or the like. However, those evaluation systems have problems such as species differences from a human, low expression levels of a drug transporter and a drug metabolic enzyme, and accumulated genetic mutations specific to cancer cells. For those reasons, it has been difficult to acquire excellent cells that can be stably tested for drug metabolism and permeability in the small intestine.

There are reports on enterocyte-like cells derived from pluripotent stem cells. Non Patent Literature 1 reports the first production of a small intestine-like tissue from human pluripotent stem cells in the world. Non Patent Literature 2 reports that small intestine stem cells capable of long-term self-replication were successfully produced from human pluripotent stem cells. Non Patent Literature 3 reports that use of a glycogen synthase kinase 3β (GSK-3β) inhibitor 6-bromoindirubin-3'-oxime (BIO), a γ-secretase inhibitor N-[(3,5-difluorophenyl)acetyl]-L-alanyl-2-phenyl]glycine-1,1-dimethylethyl ester (DAPT), or the like enabled promotion of induction of differentiation from murine and human pluripotent stem cells into small intestine-lineage cells. Combined use of BIO and DAPT allows efficient induction of differentiation into caudal type homeobox 2 (CDX2)-positive cells. CDX2 is a master transcription factor that is expressed in any of the hindgut, small intestine stem cells, intestinal progenitor cells, and enterocytes and controls intestinal differentiation. Non Patent Literature 4 reports that human pluripotent stem cells were induced to differentiate to produce enterocyte-like cells expressing small intestinal markers, such as sucrase isomaltase (SI), SLC15A1/PEPT1, and leucine-rich repeat containing G protein-coupled receptor 5 (LGR5). The enterocyte-like cells are capable of incorporating a dipeptide β-Ala-Lys-N-7-amino-4-methylcoumarin-3-acetic acid (β-Ala-Lys-AMCA), but have an extremely low expression of the drug metabolic enzyme CYP3A4 as compared to that of a human small intestine (about 1/500). Non Patent Literature 5 reports that human pluripotent stem cells were induced to differentiate to produce enterocyte-like cells expressing small intestinal markers, such as SI, villin 1 (VIL1), SLC15A1/PEPT1, and ABCG2/BCRP. Further, Non Patent Literature 6 reports that the enterocyte-like cells had SLC15A1/PEPT1 activity and ABCG2/BCRP activity. However, but expressions of the drug transporters P-gp/ABCB1/MDR1 are extremely low as compared to those of a human small intestine (about 1/100).

Patent Literature 1 discloses that in a step of inducing differentiation from intestinal progenitor cells into enterocyte-like cells, use of a medium containing LY2090314 and vitamin D3 instead of BIO and DAPT leads to more effective induction of differentiation into enterocyte-like cells (Patent Literature 1).

There are reports on a method including dispersing intestinal organoids to prepare single cells and culturing the single cells as a monolayer on an extracellular matrix (Patent Literature 2 and Non Patent Literature 7). However, the cells disclosed in Patent Literature 1 are cells for infection and propagation of a human diarrhea virus, and the cells disclosed in Non Patent Literature 7 are cells for examining an influence on a barrier function in the large bowel by enteric bacteria (*Klebsiella pneumoniae*); both are not cells used for pharmacokinetic evaluation, and none of the literatures mentions the expression of a drug metabolic enzyme or a drug transporter at all. Patent Literature 3 discloses that organoid-derived cells were cultured as a monolayer to produce excellent enterocyte-like cells that expressed drug metabolic enzymes (each member of the cytochrome P450 family), drug transporters (SLC15A1/PEPT1 and P-gp/ABCB1/MDR1), and the like belonging to enterocytes and also had a tight junction function.

There have been needs for enterocyte-like cells that are applicable to safety evaluation of a drug and has higher functionality, and a method of producing the same.

Citation List

Non Patent Literature

[NPL 1] Nature, 2011 Feb 3; 470(7332): 105-9
[NPL 2] Stem Cell Reports, 2014 Jun 3; 2(6): 838-52
[NPL 3] Stem Cells, 2013 Jun; 31(6): 1086-96
[NPL 4] Drug Metab Pharmacokinet, 2014; 29(1): 44-51
[NPL 5] Drug Metab Dispo, 2015; 43(4): 603-610
[NPL 6] Drug Metab Dispo, 2016 Oct; 44(10):0. doi: 10.1124/dmd. 116.069336. Epub 2016 Jul 14.
[NPL 7] Nature Microbiology, 2019 March; Vol. 4: 492-503

Patent Literature

[PTL 1] WO 2020/262492 A1
[PTL 2] WO 2018/038042 A1
[PTL 3] JP 2021-122208 A
[PTL 4] WO 2018/20714 A1

Summary of Invention

Technical Problem

Human primary enterocytes are not easily available, and the obtained cells have had problems of differences in properties due to individual variability. As model cells for an in vitro evaluation system of small intestinal absorption, Caco-2 cells, which are capable of forming a robust tight junction and can provide predictive permeation of a drug in the small intestine, have been in practical use. However, the Caco-2 cells have problems in that the cells are derived from cancer cells, and little expression of the drug metabolic enzyme CYP3A4 unlike human enterocytes. There have been strong demands for excellent model cells for an evaluation system that can be stably tested for drug metabolism and permeability. While studies have been made on a method of selectively inducing differentiation from pluripotent stem cells into enterocyte-like cells, there have been needs for provision of enterocyte-like cell population having higher functionality, and a method of efficiently producing cells.

Solution to Problem

To solve the above-mentioned problems, the inventors of the present invention have further investigated a culture liquid and a culture time on the basis of a related-art method of producing enterocyte-like cells, and have consequently found that pluripotent stem cells can be more effectively induced to differentiate into enterocyte-like cells having high functionality by inducing differentiation from the pluripotent stem cells into definitive endoderm cells, and then producing intestinal progenitor cells with devising a culture ingredient. Further, the inventors have also investigated a method of culturing enterocyte-like cells produced by the induction of differentiation, have consequently found that a cell population of enterocyte-like cells is more effectively obtained by seeding and culturing the cells in a base material for organoid production or a base material for two-dimensional culture, and thus have completed the present invention.

That is, the present invention includes the following.

1. A method of inducing differentiation from pluripotent stem cells into enterocyte-like cells, the method including the following steps (1) and (2):

(1) a step of inducing differentiation from pluripotent stem cells into definitive endoderm cells; and
(2) a step of culturing the definitive endoderm cells in a system containing CHIR99021, followed by induction of differentiation into intestinal progenitor cells.

2. The method of inducing differentiation according to the above-mentioned item 1, wherein the step (2) includes the following step (2a) and step (2b):

(2a) a step of culturing the definitive endoderm cells in a system containing CHIR99021 and further containing one or more kinds selected from LY2090314 and retinoic acid; and

(2b) a step of further culturing the definitive endoderm cells in a system containing two or more kinds selected from CHIR99021, retinoic acid, and LY2090314.

3. The method of inducing differentiation into enterocyte-like cells according to the above-mentioned item 2, wherein the step (2a) is performed in a system containing CHIR99021 and retinoic acid, and the step (2b) is performed in a system containing CHIR99021 and further containing retinoic acid and/or LY2090314.

4. The method of inducing differentiation into enterocyte-like cells according to the above-mentioned item 2, wherein the step (2a) is performed in a system containing LY2090314 and CHIR99021, and the step (2b) is performed in a system containing CHIR99021 and retinoic acid.

5. The method of inducing differentiation into enterocyte-like cells according to the above-mentioned item 2, wherein the step (2a) is performed in a system containing LY2090314, CHIR99021, and retinoic acid, and the step (2b) is performed in a system containing CHIR99021 and retinoic acid.

6. A method of inducing differentiation from pluripotent stem cells into enterocyte-like cells, the method including the following steps (1) to (3):

(1) a step of introducing differentiation from pluripotent stem cells into definitive endoderm cells;

(2') a step of culturing the definitive endoderm cells in a system containing LY2090314 and/or CHIR99021, followed by induction of differentiation into intestinal progenitor cells; and

(3) a step of culturing the intestinal progenitor cells in a system containing LY2090314 and/or a MEK inhibitor.

7. The method of introducing differentiation according to the above-mentioned item 6, wherein the step (2') includes the following step (2'a) and step (2'b):

(2'a) a step of culturing the definitive endoderm cells in a system containing LY2090314 for 2 days; and

(2'b) a step of culturing the definitive endoderm cells in a system containing LY2090314 or CHIR99021 for 2 days.

8. The method of inducing differentiation according to the above-mentioned item 6, wherein the step (3) includes the following step (3a) and step (3b):

(3a) a step of culturing the intestinal progenitor cells in a system containing LY2090314 for 10 days; and

(3b) a step of culturing the intestinal progenitor cells in a system containing LY2090314 and a MEK inhibitor for 10 days.

9. The method of inducing differentiation according to the above-mentioned item 6 or 8, wherein the MEK inhibitor is PD0325901.

10. A method of producing a cell population of enterocyte-like cells derived from pluripotent stem cells, the method including the following steps (I) to (IV):

(I) a step of inducing differentiation of pluripotent stem cells into definitive endoderm cells;

(II) a step of culturing the definitive endoderm cells obtained by the induction of differentiation, in a system containing LY2090314 and/or CHIR99021, followed by induction of differentiation into intestinal progenitor cells;

(III) a step of culturing the intestinal progenitor cells in a system containing LY2090314 and/or a MEK inhibitor to induce differentiation into the enterocyte-like cells; and

(IV) a step of dissociating the enterocyte-like cells obtained by the induction of differentiation into single cells, followed by seeding and culturing the enterocyte-like cells in a base material for organoid production to produce a cell population of enterocyte-like cells.

11. The method of producing a cell population according to the above-mentioned item 10, wherein a term of the steps (I) to (III) is from 17 days to 27 days after a start of the induction of differentiation of the pluripotent stem cells.

12. The method of producing a cell population according to the above-mentioned item 10, wherein the step of seeding and culturing the enterocyte-like cells in a base material for organoid production in the step (IV) includes a step of culturing the enterocyte-like cells in a medium containing at least Wnt3A, R-spondin, and Noggin.

13. The method of producing a cell population according to the above-mentioned item 10, further including the following step (V) after the seeding and culturing of the enterocyte-like cells in the base material for organoid production in the step (IV):

(V) a step of further dissociating the cell population of enterocyte-like cells produced by the seeding in the base material for organoid production into single cells, followed by seeding and culturing in a base material for two-dimensional culture to produce a cell population of enterocyte-like cells.

14. The method of producing a cell population according to the above-mentioned item 13, wherein the step of seeding and culturing the enterocyte-like cells in the base material for two-dimensional culture in the step (V) includes a step of culturing the enterocyte-like cells in a medium containing at least MEM NEAA (product name), B27 supplement (product name), Knockout Serum Replacement (product name), SB431542, PD0325901, LY20903144, and Y-27632.

15. Enterocyte-like cells produced by the method of any one of the above-mentioned items 10 to 14.

16. A medium for inducing differentiation from definitive endoderm cells into intestinal progenitor cells, including CHIR99021, retinoic acid, and LY2090314.

Advantageous Effects of Invention

The enterocyte-like cells of the present invention maintain high activities of drug metabolic enzymes as well as gene expressions of the drug metabolic enzymes. Thus, the enterocyte-like cells can be effectively utilized for in vitro pharmacokinetic evaluation. The enterocyte-like cells of the present invention are also applicable to an induction test of CYP3A4. Further, the enterocyte-like cells produced by the method of the present invention and a cell population thereof can be cryopreserved and passaged, can provide a large amount of cells with excellent performance in the same lot, and can semipermanently supply enterocyte-like cells with constant quality.

Brief Description of Drawings

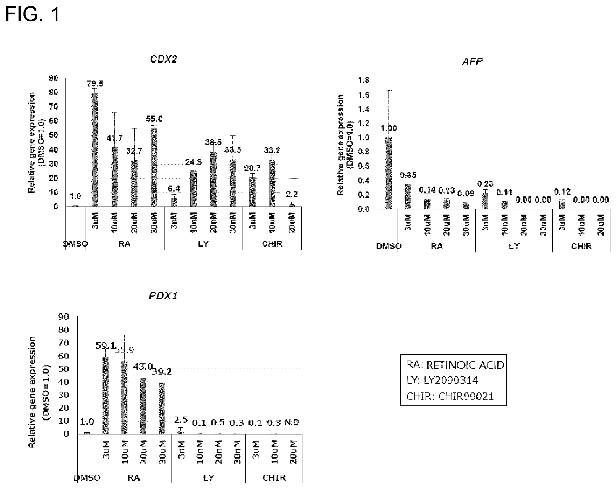

FIGS. 1 show results of examining differentiation-inducing effects of various concentrations of retinoic acid or GSK3β inhibitors (LY2090314 and CHIR99021) in a step of inducing differentiation from definitive endoderm cells into intestinal progenitor cells in a process of producing enterocyte-like cells (ELCs), by expression levels of the respective genes of CDX2, alpha fetoprotein (AFP), and pancreatic and duodenal homeobox 1 (PDX1). (Example 1)

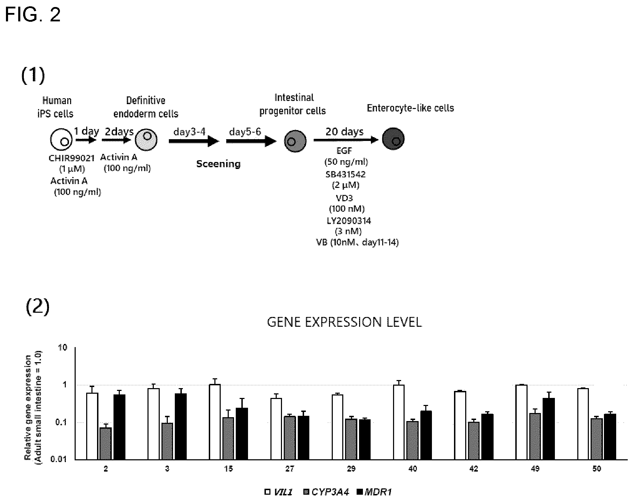

FIGS. 2 show results of examining differentiation-inducing effects of the respective combinations of retinoic acid and GSK3β inhibitors (LY2090314 and CHIR99021) in a step of inducing differentiation from definitive endoderm cells into intestinal progenitor cells in a process of producing enterocyte-like cells (ELCs). FIG. 2(1) is an illustration of a protocol, and FIG. 2(2) shows expression levels of each gene of VIL1, CYP3A4, and MDR1. (Example 2)

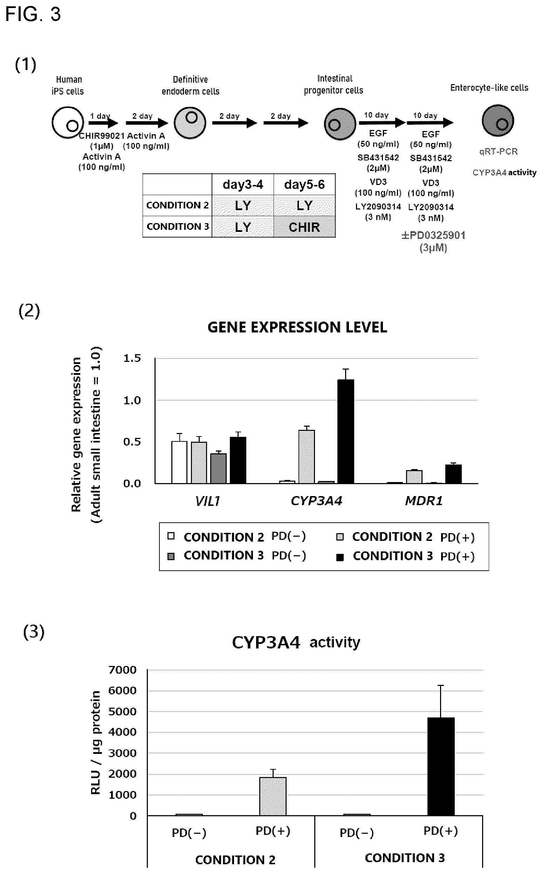

FIGS. 3 show results of examining a differentiation-inducing effect of a mitogen-activated protein kinase kinase (MEK) inhibitor (PD0325901) in a step of inducing differentiation from intestinal progenitor cells into enterocyte-like cells (ELCs) in a process of producing enterocyte-like cells (ELCs). FIG. 3(1) is an illustration of a protocol, FIG. 3(2) shows expression levels of each gene of VIL1, CYP3A4, and MDR1, and FIG. 3(3) shows CYP3A4 activity. (Example 3)

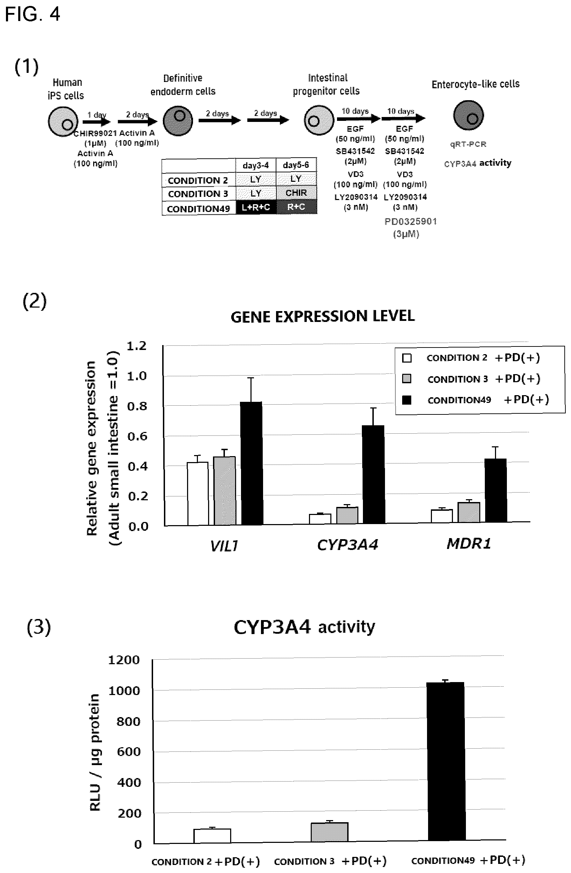

FIGS. 4 show results of examining a differentiation-inducing effect of a MEK inhibitor (PD0325901) in a step of inducing differentiation from intestinal progenitor cells into enterocyte-like cells (ELCs) in a process of producing enterocyte-like cells (ELCs). The examination was made for an effect of the MEK inhibitor under each condition of combinations of 10 μM retinoic acid, 20 nM LY2090314, and 10 μM CHIR99021 in a step of inducing differentiation from definitive endoderm cells into intestinal progenitor cells. FIG. 4(1) is an illustration of a protocol, FIG. 4(2) shows expression levels of each gene of VIL1, CYP3A4, and MDR1, and FIG. 4(3) shows CYP3A4 activity. (Example 4)

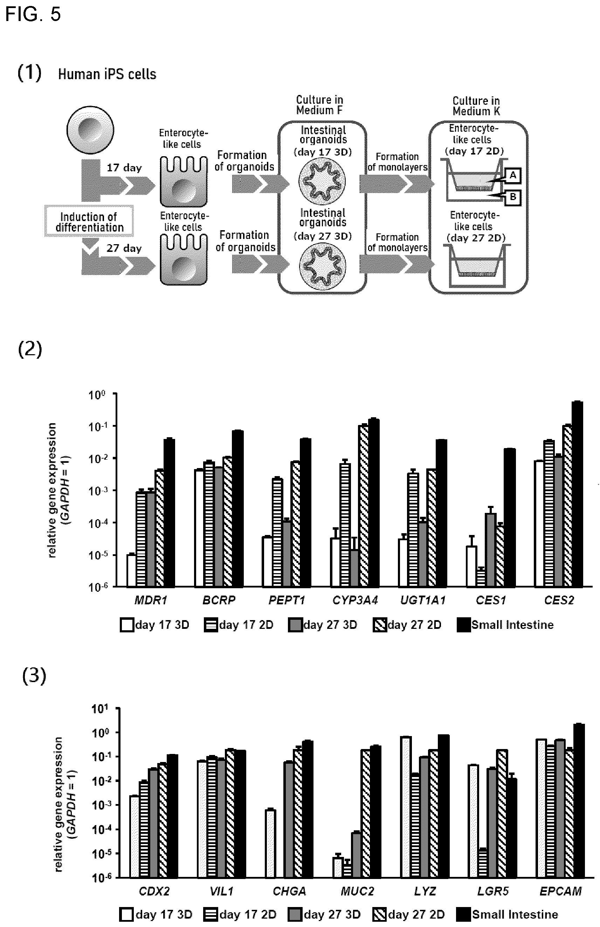

FIGS. 5 show results of analyzing gene expression levels of pharmacokinetics-related molecules and intestinal differentiation markers for ELC organoids and ELC monolayers produced from enterocyte-like cells (ELCs). FIG. 5(1) is an illustration of a protocol for producing ELC organoids and ELC monolayers, FIG. 5(2) shows each gene expression level of pharmacokinetics-related molecules (MDR1, BCRP, PEPT1, CYP3A4, UGT1A1, CES1, and CES2), and FIG. 5(3) shows each gene expression level of intestinal differentiation markers (CDX2, VIL1, CHGA, MUC2, LYZ, LGR5, and EPCAM). (Example 5)

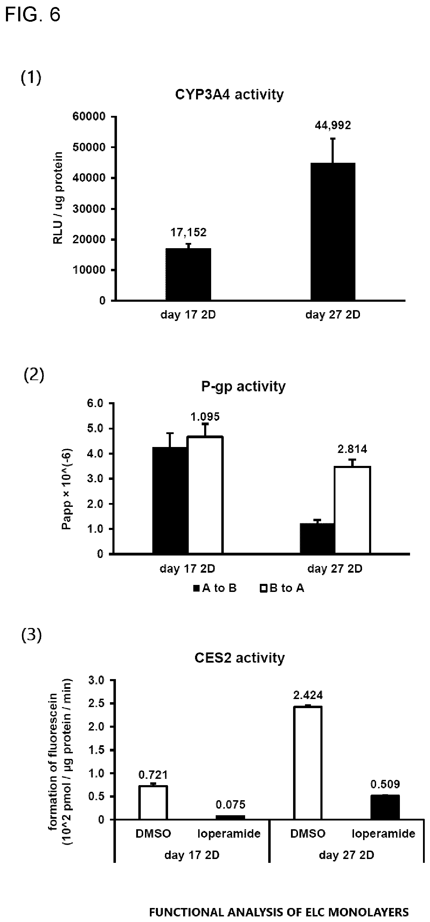

FIGS. 6 show various activities of the ELC monolayers (2D) produced in Example 5. FIG. 6(1) shows results of measuring CYP3A4 activity, FIG. 6(2) shows results of measuring P-gp activity, and FIG. 6(3) shows results of measuring CES2 activity. (Example 6)

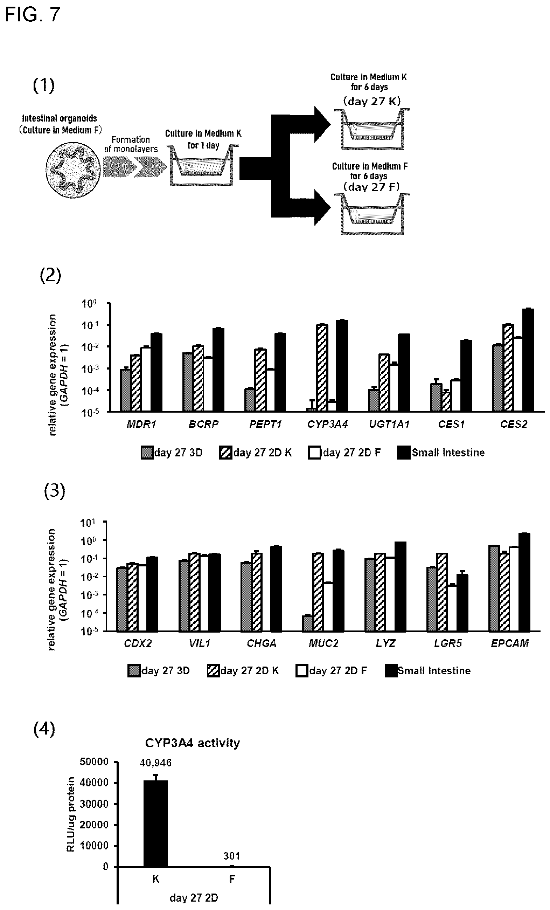

FIGS. 7 show results of evaluating ELC organoids, and an ELC monolayer cultured in a medium K or a medium F. FIG. 7(1) is an illustration of a protocol, FIG. 7(2) shows gene expression levels of pharmacokinetics-related molecules (MDR1, BCRP, PEPT1, CYP3A4, UGT1A1, CES1, and CES2), FIG. 7(3) shows gene expression levels of intestinal differentiation markers (CDX2, VIL1, CHGA, MUC2, LYZ, LGR5, and EPCAM), and FIG. 7(4) shows CYP3A4 activity. (Example 7)

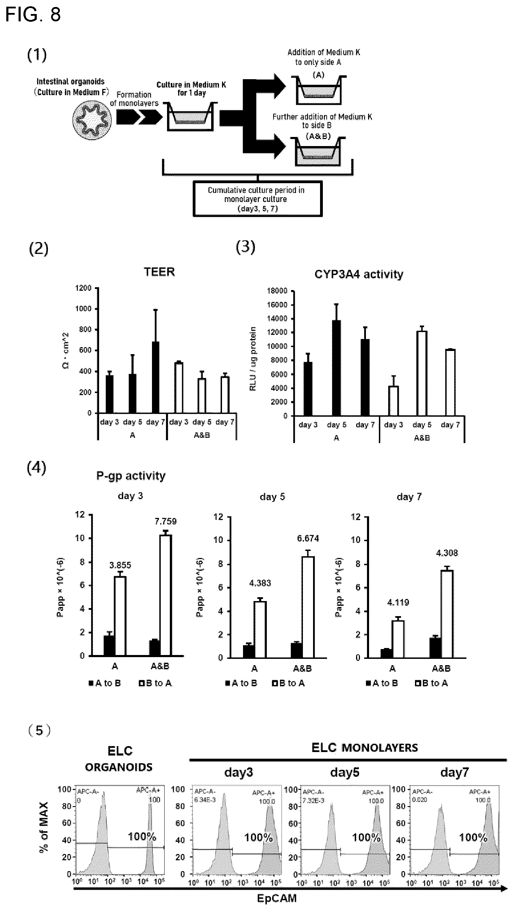

FIGS. 8 show results of investigating a culture period and a culture condition of an ELC monolayer. FIG. 8(1) is an illustration of a protocol, FIG. 8(2) shows transepithelial electrical resistance values (TEER), and FIG. 8(3) shows CYP3A4 activity. FIG. 8(4) shows P-gp activity. FIG. 8(5) shows rates of cells positive for EpCAM, which is an intestinal epithelial cell marker. (Example 8)

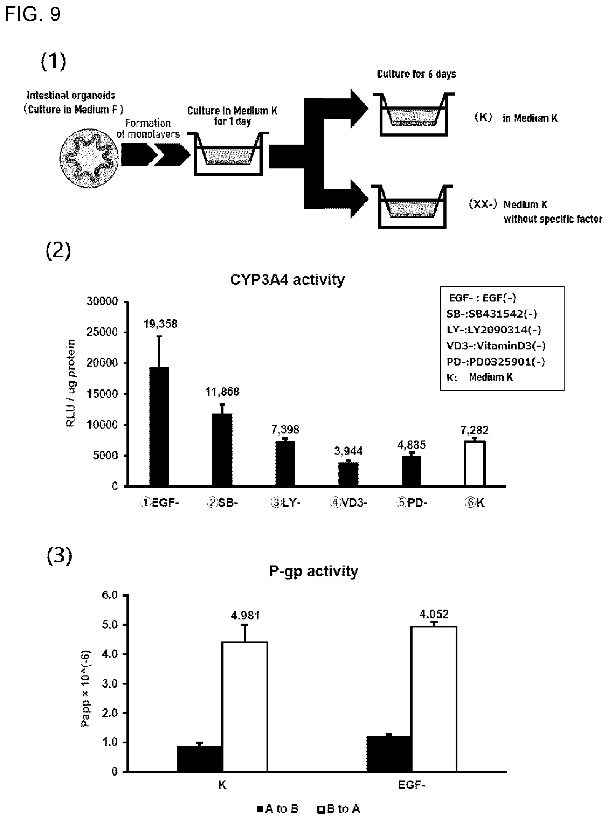

FIGS. 9 show results of investigating an influence of a humoral factor in culture of an ELC monolayer. FIG. 9(1) is an illustration of a protocol, FIG. 9(2) shows CYP3A4 activity, and FIG. 9(3) shows P-gp activity. (Example 9)

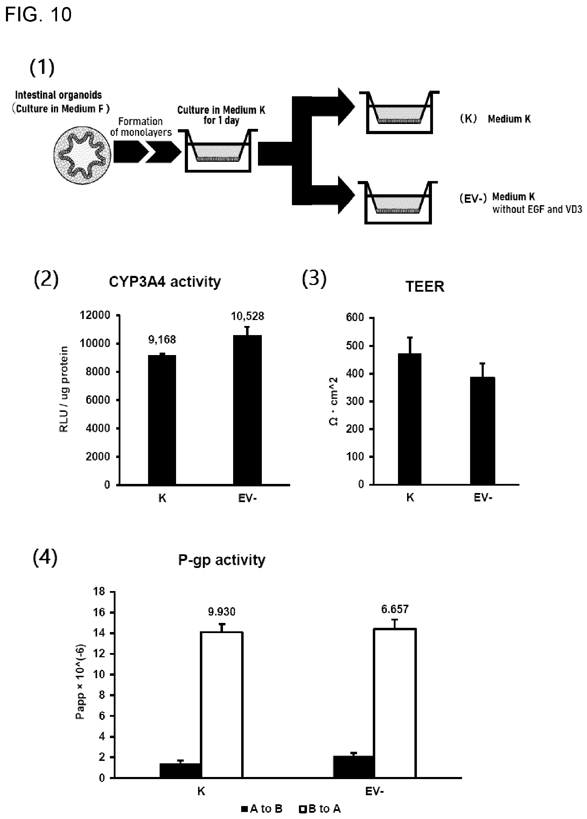

FIGS. 10 show results of investigating an influence of a humoral factor in culture of an ELC monolayer. FIG. 10(1) is an illustration of a protocol, FIG. 10(2) shows CYP3A4 activity, and FIG. 10(3) shows transepithelial electrical resistance values (TEER). FIG. 10(4) shows P-gp activity. (Example 10)

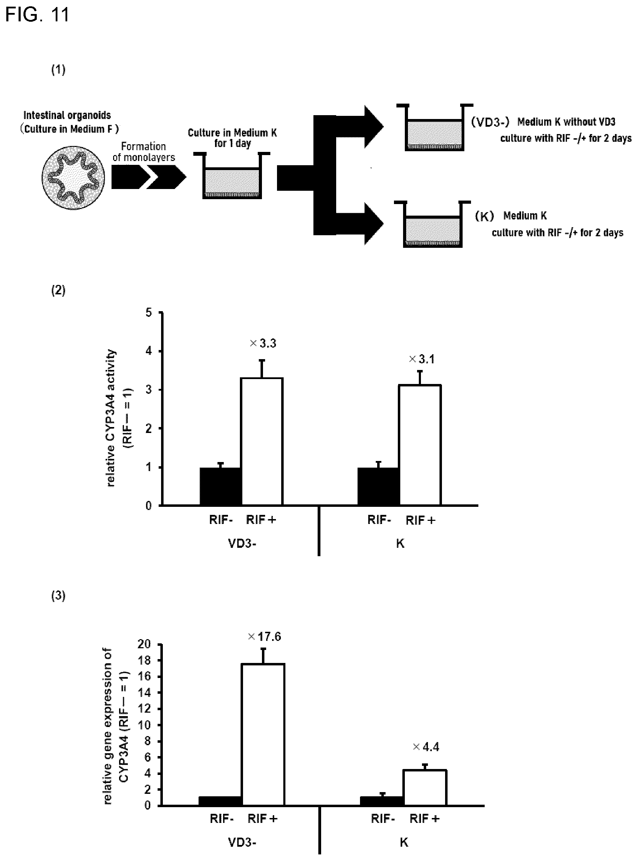

FIGS. 11 show results of measuring a membrane function of an ELC monolayer. FIG. 11(1) is an illustration of a protocol, FIG. 11(2) shows CYP3A4 activity, and FIG. 11(3) shows gene expression levels of CYP3A4. (Example 11)

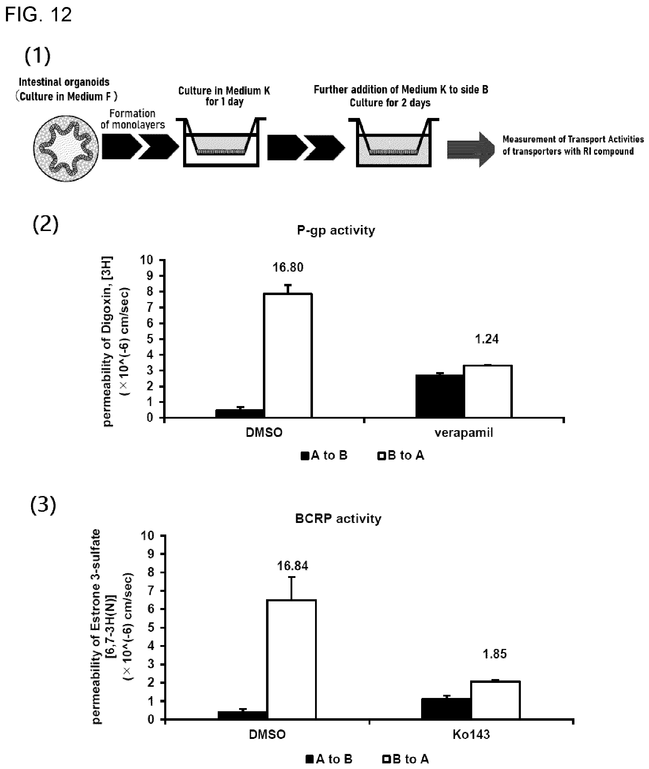

FIGS. 12 show results of measuring a membrane function of an ELC monolayer. FIG. 12(1) is an illustration of a protocol, FIG. 12(2) shows P-gp activity, and FIG. 12(3) shows BCRP activity. (Example 12)

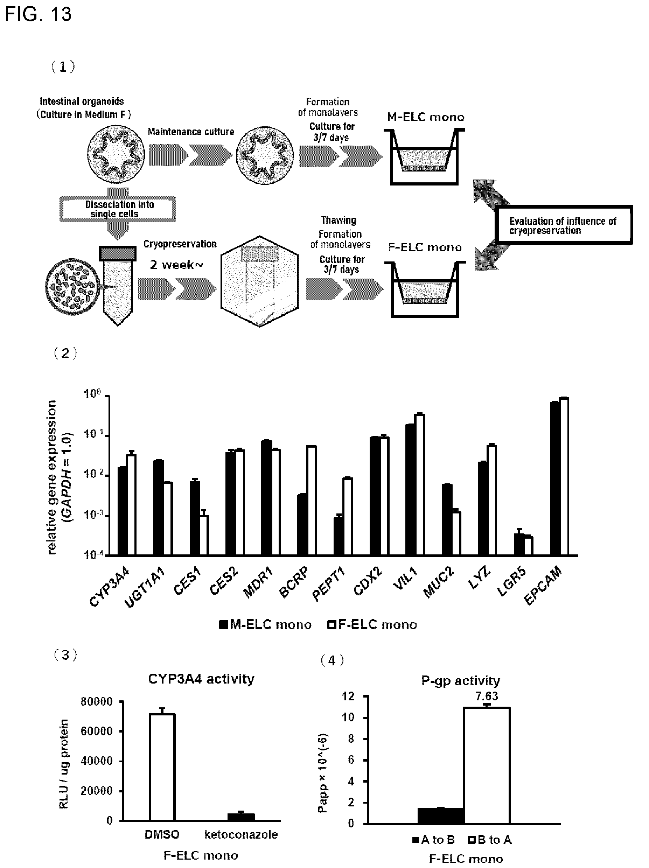

FIGS. 13 show results of analyzing gene expression levels of pharmacokinetics-related molecules and intestinal differentiation markers in an ELC monolayer produced from cryopreserved ELC organoids. FIG. 13(1) is an illustration of a protocol, FIG. 13(2) shows expression levels of various marker genes, FIG. 13(3) shows CYP3A4 activity, and FIG. 13(4) shows P-gp activity. (Example 13)

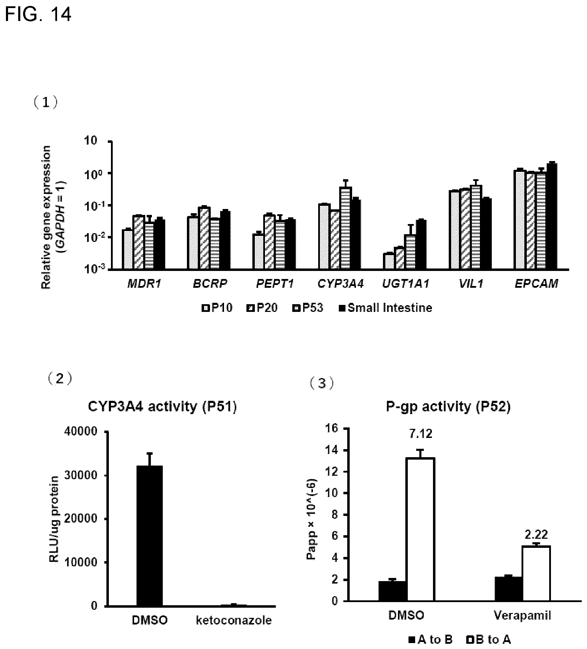

FIGS. 14 show results of investigating an influence of long-term culture of ELC organoids on a function of an ELC monolayer. FIG. 14(1) shows gene expression levels of pharmacokinetics-related molecules (MDR1, BCRP, PEPT1, CYP3A4, UGT1A1, CES1, and CES2) and intestinal differentiation markers (CDX2, VIL1, MUC2, LYZ, LGR5, and EPCAM), FIG. 14(2) shows CYP3A4 activity, and FIG. 14 (3) shows P-gp activity. (Example 14)

Description of Embodiments

The present invention relates to a method of inducing differentiation from pluripotent stem cells into enterocyte-like cells, and to enterocyte-like cells obtained by the induction of differentiation. The present invention also relates to a cell population of the enterocyte-like cells. The present invention also relates to a method of evaluating drug toxicity and/or a method of evaluating of pharmacokinetics with use of the enterocyte-like cells, and to a medium composition and a medium preparation kit for inducing differentiation into the enterocyte-like cells.

(Pluripotent Stem Cells)

The pluripotent stem cells as used herein only need to be undifferentiated cells having pluripotency and/or a self-replication ability, and are not particularly limited, but include pluripotent stem cells, such as induced pluripotent stem cells (iPS cells) or embryonic stem cells (ES cells). Of those, iPS cells are particularly suitable.

The iPS cells refer to induced pluripotent stem cells having pluripotency and proliferative capacity similar to ES cells. The iPS cells were generated by inducing reprogramming of differentiated cells without use of fertilized eggs, surplus embryos, or ES cells by introducing several kinds of genes into somatic cells, and were established from mouse fibroblasts in 2006 for the first time in the world (Cell 126: 663-676, 2006). Further, it has been reported that human iPS cells were successfully established by introducing four human genes OCT3/4, SOX2, KLF4, and C-MYC homologous to the four genes used in establishing mouse iPS cells into fibroblasts derived from humans (Cell 131: 861-872, 2007). The iPS cells to be used in the present invention may be iPS cells generated by such a known method per se as the above-mentioned method or may be iPS cells to be generated by a novel method developed in the future. Specifically, for example, Tic (JCRB1331) may be used as a human iPS cell line. YOW and YEM (Natl. Acad. Sci. USA, 111(47): 16772-7, 2014) may also be used.

The ES cells are pluripotent stem cells obtained by transferring a cell mass called an inner cell mass, which is generally present in the embryo at the blastocyst stage, to in vitro culture, followed by isolation as a population of undifferentiated stem cells from the culture. The ES cells were established as a cell line having pluripotency in mice by M.J. Evans & M.H. Kaufman (Nature, 292, 154, 1981), and then by G.R. Martin (Natl. Acad. Sci. USA, 78, 7634, 1981). There are many human-derived ES cell lines already established and these cell lines are available from, for example, ES Cell International, Wisconsin Alumni Research Foundation, National Stem Cell Bank (NSCB), and the like. The ES cells are generally established by culturing early embryos. The ES cells may also be generated from early embryos containing the nuclei of somatic cells transplanted. Alternatively, there is a method involving generating a cell structure like the embryo at the blastocyst stage by transplanting the cell nuclei of a desired animal into cell vesicles (cytoplasts or ooplastoids) generated by dividing egg cells or denucleated egg cells of a foreign animal into a plurality of pieces and generating ES cells based on the cell structure. In addition, there have been reported an attempt to generate ES cells by developing parthenogenetic embryos to the phase similar to the blastocyst stage and generating ES cells from the embryos and a method involving generating ES cells having genetic information on the nuclei of somatic cells by fusion of ES cells and somatic cells. The ES cells to be used in the present invention may be ES cells generated by such a known method per se as the above-mentioned method or may be ES cells to be generated by a novel method developed in the future. For example, a human ES cell line (KhES-1, KhES-2, KhES-3, H1, or H9) may be used as the ES cells.

(Definitive Endoderm Cells)

Embryonic definitive endoderm cells particularly refer to, among definitive endoderm cells, cells that can differentiate into intestine-derived organs, such as esophagus, stomach, whole intestine including small intestine and large intestine, as well as lung, liver, thymus, parathyroid gland, thyroid gland, gallbladder, and pancreas. The term "definitive endoderm cells" as used herein refers to definitive endoderm cells produced from pluripotent stem cells used in the method of inducing differentiation of the present invention. Examples of a gene expression marker of definitive endoderm include SRY-box transcription factor 17 (SOX17), forkhead box A2 (FOXA2), and C-X-C motif chemokine receptor 4 (CXCR4).

(Intestinal Progenitor Cells)

The term "intestinal progenitor cells" as used herein refers to cells before introduction of differentiation into enterocyte-like cells, and has the same meaning as, for example, small intestine progenitor cells. A gene expression marker of the intestinal progenitor cells is, for example, CDX2.

(Enterocyte-like Cells)

Enterocytes mean cells in a tissue covering a luminal surface of the small intestine, and encompass absorbing cells, endocrine cells, and crypt cells (mucous gland cells, serous acid cells, and stem cells) of the small intestine. The term "enterocyte-like cells" as used herein is distinguished from enterocytes directly harvested from a living body, and means cells produced by the method of inducing differentiation or treatment for inducing differentiation of the present invention. Examples of a marker that is an indicator of enterocytes include influx transporters, drug metabolic enzymes, and efflux transporters. Examples of the influx transporters include a peptide transporter PEPT1 and an organic anion transporter OATP2B1. An example of the drug metabolic enzymes is cytochrome P450 (CYP), and the CYP is, for example, CYP3A4, CYP1A2, CYP2B6, CYP2C8, CYP2C9, CYP2C19, CYP2D6, or CYP2E1, preferably CYP3A4, CYP1A2, CYP2B6, CYP2C9, CYP2C19, CYP2D6, or CYP2E1, most preferably CYP3A4. Examples of the efflux transporters include P-gp and ABCG2/BCRP. The gene encoding P-gp is called MDR1, and the gene encoding BCRP is called ABCG2. P-gp is a phosphorylated protein having a molecular weight of about 180,000, and is present on a cell membrane and is responsible for efflux of a cytotoxic compound and the like out of cells. Other examples include VIL1, CDX2, SI, SLC15A1/PEPT1, LGR5, fatty acid binding protein 2 (FABP2), intestine specific homeobox (ISX), lactase, solute carrier family 2 member 2 (SLC2A2)/glucose transporter type 2 (Glut2), and zonula (zona) occludens 1 protein (Zo-1 protein, also called tight-junction protein-1 (Tjp-1)).

(Induction of Differentiation from Pluripotent Stem Cells into Enterocyte-like Cells)

In the process of inducing differentiation from pluripotent stem cells into enterocyte-like cells of the present invention, the induction of differentiation can be performed (1) from pluripotent stem cells into definitive endoderm cells, (2) definitive endoderm cells into intestinal progenitor cells, and (3) from intestinal progenitor cells into enterocyte-like cells.

I. Basal Medium for Induction of Differentiation

In the method of inducing differentiation of the present invention, as a medium for maintaining undifferentiation of human ES/iPS cells, various medium for maintaining stem cells may be used such as ReproStem (product name), iPSellon (product name), Essential 8 (product name), TeSR-E8 (product name), StemFit™ AK03N (product name), and StemFit™ AK02N (product name). In the method of inducing differentiation of the present invention, for example, a medium A, a medium B, and a medium C described below may each be used as a basal medium in a process of differentiation from pluripotent stem cells into enterocyte-like cells. In terms of each reagent to be used, a manufacturer and a supplier are not limited to those of reagents specifically described below, and any reagent may be employed as long as the reagent can exert a comparable function. Among all culture steps, after a passage operation, a Rho kinase (ROCK) inhibitor may be used. The passage operation may be performed by a method known per se, specifically by: loading cultured cells into a liquid containing at least one of, for example, a protease, such as trypsin, collagenase, or dispase I, EDTA, or EGTA, for example, into TrypLE Select™; dissociating the cells into single cells through use of, for example, filtration, a centrifuge, or pipetting; suspending the cells dissociated into single cells in a fresh medium; and seeding the cells in a culture medium or the like. For example, Y27632, HA1100, HA1077, thiazovivin, and GSK429286 may each be used as the Rho kinase inhibitor, but the inhibitor is not limited thereto. The Rho kinase inhibitor may be added to a medium for a certain period after passage or for a certain period after thawing of cells, specifically for a whole or partial period of culture after thawing of small intestinal epithelial progenitor cells, and may be added to a medium for from 1 day to 5 days after the thawing of the cells, preferably for from 1 day to 3 days after the thawing, particularly preferably for 1 day after the thawing. A concentration and an application period of the Rho kinase inhibitor may be appropriately selected as required. For example, in use of Y27632, the inhibitor may be used at a final concentration of, for example, from 0.1 $\mu$M to 100 $\mu$M, preferably from 1 nM to 10 nM in a medium.

(1) Medium for Inducing Differentiation from Pluripotent Stem Cells into Definitive Endoderm Cells (Medium A) RPMI: RPMI 1640 medium (Sigma-Aldrich Co. LLC)

2 mM Gln: 2 mM L-glutamine (FUJIFILM Wako Pure Chemical Corporation)
B27: 0.5$\times$ B27 Supplement Minus Vitamin A (Thermo Fisher Scientific Inc.)
P/S: Penicillin-Streptomycin Solution ($\times$100) (FUJIFILM Wako Pure Chemical Corporation)
10% KSR: 10% knockout serum replacement (Thermo Fisher Scientific Inc.)
1% NEAA: 1% non-essential amino acid solution (Thermo Fisher Scientific Inc.)

(2) Medium for Inducing Differentiation from Definitive Endoderm Cells into Intestinal Progenitor Cells (Medium B)

DMEM: DMEM-High Glucose medium (FUJIFILM Wako Pure Chemical Corporation)
1 mM Gln: 1 mM L-glutamine (FUJIFILM Wako Pure Chemical Corporation)
B27: 0.5$\times$ B27 Supplement Minus Vitamin A (Thermo Fisher Scientific Inc.)
P/S: Penicillin-Streptomycin Solution ($\times$100) (FUJIFILM Wako Pure Chemical Corporation)
10% KSR: 10% knockout serum replacement (Thermo Fisher Scientific Inc.)
1% NEAA: 1% non-essential amino acid solution (Thermo Fisher Scientific Inc.)

(3) Medium for Inducing Differentiation from Intestinal Progenitor Cells into Enterocyte-like Cells (Medium C)

DMEM: DMEM-High Glucose medium (FUJIFILM Wako Pure Chemical Corporation)
1 mM Gln: 1 mM L-glutamine (FUJIFILM Wako Pure Chemical Corporation)
B27: 0.5$\times$ B27 Supplement Minus Vitamin A (Thermo Fisher Scientific Inc.)
P/S: Penicillin-Streptomycin Solution ($\times$100) (FUJIFILM Wako Pure Chemical Corporation)
10% KSR: 10% knockout serum replacement (Thermo Fisher Scientific Inc.)
1% NEAA: 1% non-essential amino acid solution (Thermo Fisher Scientific Inc.)
3 nM LY: 3 nM LY2090314 (MedChemExpress LLC)
2 $\mu$M SB: 2 $\mu$M SB431542 (FUJIFILM Wako Pure Chemical Corporation)

II. Induction of Differentiation from Pluripotent Stem Cells into Enterocyte-like Cells

(1) Step of Inducing Differentiation from Pluripotent Stem Cells into Definitive Endoderm Cells

In the step (1) of inducing differentiation from pluripotent stem cells into definitive endoderm cells, a method known per se may be applied. For example, culture may be performed in a system containing Activin A in the medium A mentioned above. Activin A may be added at a concentration of from 3 ng/mL to 200 ng/mL, preferably about 100 ng/mL. When the concentration of Activin A is less than 3 ng/mL, it may be impossible to efficiently promote differentiation into definitive endoderm cells. Another compound may be further added such as a PI3K inhibitor (e.g., LY294002), a DNA methylation inhibitor (e.g., 5-aza-2'-deoxycytidine), or dimethyl sulfoxide (DMSO). The induction of differentiation from pluripotent stem cells into definitive endoderm cells is performed until day 5, most preferably until day 3 after the start of the induction of differentiation.

In particular, on days 0 to 1 after the start of the culture of the pluripotent stem cells, the culture may be performed in a system containing a GSK3$\beta$ inhibitor in the above-mentioned culture system. For example, CHIR99021 may be used as the GSK3$\beta$ inhibitor. More specifically, the culture system may contain 0.01 $\mu$M to 10 $\mu$M, preferably about 1 $\mu$M of CHIR99021. The GSK3$\beta$ inhibitor may be eliminated from day 2 after the start of the induction of the differentiation to formation of definitive endoderm cells.

(2) Step of Inducing Differentiation from Definitive Endoderm Cells into Intestinal Progenitor Cells

In the step (2) of inducing differentiation into intestinal progenitor cells, it is necessary to perform culture in a system further containing, for example, CHIR99021, among the GSK3$\beta$ inhibitors, in addition to the above-mentioned medium B. CHIR99021 may be added to a culture system at a concentration of from 3 μM to 100 μM, preferably from 5 μM to 20 μM, most preferably 10 μM. When the concentration of CHIR99021 is less than 3 μM, it may be impossible to efficiently promote differentiation. The timing of adding CHIR99021 to the culture system only needs to be after the induction of differentiation from pluripotent stem cells into definitive endoderm cells, and is not particularly limited. The timing after the induction of differentiation into definitive endoderm cells may be, for example, day 3 or later after the start of the culture of pluripotent stem cells. The step (2) allows induction of differentiation from definitive endoderm cells into intestinal progenitor cells, and particularly results in significantly increased expressions of VIL1, which is a marker serving as an indicator of enterocytes, and CYP3A4 serving as a drug metabolic enzyme.

The step (2) may include the following step (2a) and step (2b). The step (2a) may be performed in a system containing CHIR99021 and retinoic acid, and the step (2b) may be performed in a system containing CHIR99021 and further containing retinoic acid and/or LY2090314. Alternatively, the step (2a) may be performed in a system containing LY2090314 and CHIR99021, and the step (2b) may be performed in a system containing CHIR99021 and retinoic acid. Further, the step (2a) may be performed in a system containing LY2090314, CHIR99021, and retinoic acid, and the step (2b) may be performed in a system containing CHIR99021 and retinoic acid.

The step (2a) and step (2b) each take from 1 day to 4 days, preferably from 1 day to 3 days, most preferably 2 days. More specifically, the step (2a) may be performed on days 3 to 4 after the start of the culture of pluripotent stem cells, and step (2b) may be performed on days 5 to 6 after the start of the culture of the pluripotent stem cells. In the step of inducing differentiation from definitive endoderm cells into intestinal progenitor cells on days 3 to 6 after the start of the culture of the pluripotent stem cells, the system of each of the combinations may be allowed to act for 4 days in total.

(3) Step of Inducing Differentiation from Intestinal Progenitor Cells into Enterocyte-like Cells

In the step (3) from intestinal progenitor cells to enterocyte-like cells, culture may be performed in a culture system known per se and containing a p38 mitogen-activated protein kinase (MAPK) inhibitor (10 μM), dexamethasone (DEX) (1 μM), epidermal growth factor (EGF) (50 ng/mL), insulin-like growth factor 1 (IGF-1) (20 ng/mL), and Wnt3a + R-spondin + Noggin (WRN: 25% Wnt3a + R-spondin + Noggin-expressing cell culture supernatant) in addition to the medium C.

In the step (3), it is preferred to perform culture in a system further containing LY2090314 and/or a MEK inhibitor in addition to the culture system known per se. MEK is crucial in differentiation and proliferation of cells, and MEK inhibitors are known as agents that exert antitumor actions. The intestinal progenitor cells produced at the step (2) may be, for example, cultured in the medium containing the above-mentioned ingredients for 10 days, undergo a passage operation, and then be further cultured for 10 days to be induced to differentiate into enterocyte-like cells. The MEK inhibitor may be added on day 7 after the start of the culture of the intestinal progenitor cells, preferably in the passage operation on day 10 after the start of the culture of the intestinal progenitor cells. That is, the culture may be performed on days 7 to 17 after the start of the culture of the pluripotent stem cells, followed by the passage operation, and then performed in the system containing the MEK inhibitor on days 17 to 27 after the start of the culture of the pluripotent stem cells. A known MEK inhibitor may be used as the MEK inhibitor, and examples thereof include Trametinib, cobimetinib, Selumetinib, Refametinib, Pimasertib, U0126, MEK 162/ARRY-162, AZD8330/ARRY-424704, GDC-0973/RG7420, GDC-0623/RG7421/XL518, CIF/RG7167/RO4987655, CK127/RG7304/RO5126766, E6201, TAK-733, PD0325901, AS703988/MSC2015103B, WX-554, CI-1040/PD184352, AS703026, PD318088, PD98059, and SL327. Of those, PD0325901 and PD184352 are preferred, and PD0325901 is particularly preferred. The concentration of the MEK inhibitor only needs to be appropriately set. For example, when PD0325901 is used, the MEK inhibitor may be added to a medium at a final concentration of, for example, from 0.1 μM to 100 μM, preferably from 0.5 μM to 50 μM, more preferably 3 μM. The culture in the system containing the MEK inhibitor results in significantly increased expressions of CYP3A4 serving as a drug metabolic enzyme, and MDR1 serving as an efflux transporter.

(Culture of Enterocyte-like Cells Produced)

The enterocyte-like cells produced through the above-mentioned steps (1) to (3) may be cultured by a method known per se or a method to be developed in the future. For example, a medium K shown in Table 1 or a medium F shown in Table 2 may be used to perform the culture.

Table 1

| Composition of medium on day 17 to day 27 after start of induction of differentiation (composition of medium k) |
| --- |
| • DMEM (High Glucose, containing L-glutamine and phenol red)<br>• L-Glutamine (final concentration: 1 mM)<br>• P/S (each final concentration: 100 U/mL / 100 μg/mL) |

(continued)

| Composition of medium on day 17 to day 27 after start of induction of differentiation (composition of medium k) |
|---|
| • MEM NEAA (final concentration: 1×, 5.6 mL) |
| • B 27 supplement (final concentration: 0.5×, 5 mL) |
| • KnockOut Serum Replacement (50 mL) |
| • Epidermal growth factor (EGF, final concentration: 50 ng/ml) |
| • SB431542 (final concentration: 2 $\mu$M) |
| • Vitamin D3 (1$\alpha$,25-dihydroxyvitamin d3, final concentration: 100 nM) |
| • LY2090314 (final concentration: 3 nM) |
| • PD0325901 (final concentration: 3 $\mu$M) |
| • Y-27632 (final concentration: 10 $\mu$M) |

Table 2

| Composition of medium F | |
|---|---|
| Component | Volume for 1 L (mL) |
| Advanced DMEM/F-12 | 335 (235) |
| HEPES (1 M) | 10 |
| Penicillin-Streptomycin Mixed Solution | 10 |
| GlutaMAX Supplement | 10 |
| B-27 Supplement | 20 |
| N-Acetyl-L-cysteine | 10 |
| 50% Aftamin-Wnt-3A serum-free conditioned medium | 500 |
| 10% HA-R-spodin1-Fc 293T conditioned medium | 100 |
| A83-01 | 1 |
| Recombinant Human Noggin (10% Noggin conditioned medium) | 1 (100) |
| human IGF-1 (insulin-like growth factor-1) | 1 |
| human FGF-basic (fibroblast growth factor-basic: FGF-2) | 0.5 |
| [Leu15]-Gastrin I | 1 |

(4) Organoids Formed of Enterocyte-like Cells (ELC Organoids)

The "ELC organoids" as used herein only need to be organoids produced from enterocyte-like cells derived from pluripotent stem cells. The term "organoid" as used herein refers to a cluster formed of organ-specific cells (a cell population or a tissue construct), and refers to cultured cells having a characteristic similar to that of an organ and proliferative capacity. An organoid is three-dimensionally formed by using a scaffold base material for cell culture (scaffold), such as a porous membrane or hydrogel (hereinafter sometimes simply referred to as "3D").

ELC organoids may be cultured with, for example, the medium F shown in Table 2 described above. The organoids may be produced by dissociating cultured or cryopreserved enterocyte-like cells into single cells with a protease such as trypsin, collagenase, or dispase I, EDTA, EGTA, or the like; recovering, washing, and centrifuging the cells; and then placing the cells in a scaffold base material for use in culture of organoids (hereinafter referred to as "organoid base material"). The organoid base material is used for promoting adhesion and proliferation of cells and retaining a three-dimensional structure, and various products thereof different in materials and pore sizes have been in practical use. The organoid base material in the present invention may have any material and structure that are known per se or developed in the future. A specific example thereof is a soluble basement membrane extracted from murine EHS sarcoma abundant in extracellular matrix (ECM) proteins including hydrogel, laminin (main component), type IV collagen, heparin sulfate proteoglycan, entactin/nidogen, and various growth factors, and for example, a Matrigel™ (Corning Incorporated) basement membrane or the like is used. A cell seeding density for producing ELC organoids only needs to lead to capability to form organoids, is not particularly limited, but is, for example, from $1\times10$ cells/40 $\mu$L droplet to $1\times10^7$ cells/40 $\mu$L droplet, preferably from $1\times10^2$ cells/40 $\mu$L droplet to $1\times10^6$ cells/40 $\mu$L droplet, most preferably about $1\times10^5$ cells/40

μL droplet. Culture may be performed with the medium F, for example, after incubation at 37°C for from 1 minute to 60 minutes, preferably from 1 minute to 30 minutes, more preferably about 15 minutes. Medium change may be appropriately performed, for example, once every 2 to 3 days.

(5) Culture of Two-dimensional Cultured Enterocyte-like Cells

Two-dimensionally-cultured enterocyte-like cells are formed two-dimensionally by dissociating the above-mentioned ELC organoids into single cells and seeding the cells in a base material for two-dimensional culture. The single cells dissociated from the ELC organoids are also referred to as "ELC organoid cells." The ELC organoid cells may be produced by a method known per se. The ELC organoids may be loaded into a liquid containing at least one of, for example, a protease, such as trypsin, collagenase, or dispase I, EDTA, or EGTA, for example, into TrypLE Select™, followed by, for example, filtration, a centrifuge, or pipetting to produce the ELC organoid cells.

The two-dimensionally-cultured enterocyte-like cells of the present invention may be produced by seeding the ELC organoid cells in a base material for two-dimensional culture and producing a monolayer. The monolayer cultured cells thus produced are hereinafter sometimes simply referred to as "2D". The base material for two-dimensional culture is not particularly limited as long as the base material allows monolayer culture of enterocyte-like cells, and example thereof may include culture base materials known per se, such as culture plates, culture dishes, and culture flasks, or any culture base material to be developed in the future. Further, culture may be performed with a plate well including an insert. As the insert, for example, a cell culture insert (Corning Incorporated) may be used. Culture base materials used in two-dimensional culture are distinguished from culture base materials used in three-dimensional culture for organoid culture.

A seeding density of the ELC organoid cells only needs to be a density that can result in formation of a monolayer after their culture, is not particularly limited, but may be, for example, from $1.0 \times 10^2$ cells/cm$^2$ to $5.0 \times 10^7$ cells/cm$^2$, preferably from $1.0 \times 10^4$ cells/cm$^2$ to $5.0 \times 10^6$ cells/cm$^2$. An environment for culture only needs to be an environment known per se, and is not particularly limited, but generally, cells may be two-dimensionally cultured under conditions at 37 $\pm 1$°C in $5 \pm 1$% CO$_2$. A culture period of the two-dimensional culture is not particularly limited as long as cells are viable, but for example, the two-dimensional culture may be performed for from 1 day to 60 days. During the culture, medium change may be appropriately carried out, and passage culture may be performed as required.

(Method of Maintaining and Storing Enterocyte-like Cells Derived from ELC Organoids)

The enterocyte-like cells of the present invention can be passaged, and also cryopreserved with a cryopreservation medium. Culture of the enterocyte-like cells of the present invention may employ the medium K shown in Table 1. Further, the medium K may be used with a medium composition component appropriately increased, reduced, or eliminated. Specifically, the culture may be performed with the medium K with elimination of EGF and/or Vitamin D3.

The timing of the cryopreservation of the enterocyte-like cells of the present invention may be in the culture of the ELC organoid cells, or in the culture of the two-dimensionally-cultured enterocyte-like cells. With regard to ways of passage culture and cell cryopreservation, such as an additive to a cryopreservation medium and a storage temperature, a method known per se or any method to be developed in the future may be applied. The cryopreserved cells thus produced may be freeze-thawed by a method known per se or any method to be developed in the future, and further undergo two-dimensional culture or three-dimensional culture, and the cells thus obtained may be used.

(Use of Enterocyte-like Cells Derived from ELC Organoids)

The enterocyte-like cells of the present invention maintain high activities of drug metabolic enzymes as well as gene expressions of the drug metabolic enzymes. Thus, the enterocyte-like cells can be effectively utilized for in vitro pharmacokinetic evaluation. The method of producing enterocyte-like cells of the present invention can provide a large amount of cells with such excellent performance in the same lot, and can semipermanently supply enterocyte-like cells with constant quality. Such enterocyte-like cells with such excellent quality and a cell population thereof can be used in pharmacokinetic evaluation and drug toxicity evaluation kits.

The present invention also encompasses a method of using the enterocyte-like cells obtained by the above-mentioned differentiation induction method in pharmacokinetic evaluation or drug toxicity evaluation. Further, the present invention also encompasses a drug toxicity evaluation method or a pharmacokinetic evaluation method, including using the enterocyte-like cells obtained by the above-mentioned method of inducing differentiation. Further, the present invention also encompasses a drug-drug interaction testing method and a drug-metabolizing enzyme induction test method, including using the enterocyte-like cells. Through the addition of a pharmaceutical candidate compound to the enterocyte-like cells obtained as described above, the pharmaceutical candidate compound can be tested and evaluated for each of drug metabolism/drug absorption, pharmacokinetics and/or drug toxicity, a drug-drug interaction, drug-metabolizing enzyme induction, and the like. The enterocyte-like cells of the present invention can also be applied to a CYP3A4 induction test. Hitherto, human primary enterocytes have been difficult to acquire, and have had a problem of differences in properties due to individual differences. However, the method of inducing differentiation of the present invention enables stable provision of excellent enterocyte-like cells.

Examples

With regard to the present invention, a method of inducing differentiation from pluripotent stem cells into enterocyte-like cells (ELCs) is specifically described for each step with reference to Reference Examples, Examples, and Experimental Examples for better understanding, but needless to say, these examples are not intended to limit the scope of the present invention. In Reference Examples, Examples, and Experimental Examples described below, the number of days after the start of the induction of differentiation means the number of days after the start of the induction of differentiation from iPS cells.

(Reference Example 1) Induction of Differentiation from Human iPS Cells into Definitive Endoderm Cells

Pluripotent stem cells differentiate into enterocyte-like cells (ELCs) via definitive endoderm cells and intestinal progenitor cells. In Reference Example 1, description is made of maintenance culture of human iPS cells, and an operation of inducing differentiation from human iPS cells into definitive endoderm cells. In Examples described below, the maintenance culture of human iPS cells and the operation of inducing differentiation from human iPS cells into definitive endoderm cells are not limited to those described below and may be appropriately modified, as long as the culture and the operation enable induction of differentiation into definitive endoderm cells.

•Maintenance Culture of Human iPS Cells

The human iPS cell line, YOW-iPS cells were maintained with AK02N medium (StemFit AK02N medium, Ajinomoto Co., Inc.) supplemented with 0.1 $\mu$g/cm$^2$ iMatrix-511 (recombinant human laminin 511 E8 fragments, Nippi, Incorporated). Passage was performed every 6 days. A trypsin reagent (TrypLE Select Enzyme, Thermo Fisher Scientific Inc.) was allowed to act on a colony of the human iPS cells at 37°C for 6 minutes to detach the cells. The cells were seeded at $1\times10^3$ cells/cm$^2$ in AK02N medium (Ajinomoto Co., Inc.) containing iMatrix-511 and 10 $\mu$M Y-27632 (FUJIFILM Wako Pure Chemical Corporation).

• Induction of Differentiation from Human iPS Cells into Definitive Endoderm Cells

Undifferentiated human iPS cells were detached with a trypsin reagent (Thermo Fisher Scientific Inc.), and seeded at $6\times10^5$ cells/well on a 12-well plate for cell culture (Sumitomo Bakelite Co., Ltd.) coated by loading, into each well, RPMI 1640 medium (Sigma-Aldrich Co. LLC), which Matrigel (Corning Incorporated) had been added at 2 vol%, and performing incubation at 37°C for 1 hour. AK02N medium (Ajinomoto Co., Inc.) containing 10 $\mu$M Y-27632 (FUJIFILM Wako Pure Chemical Corporation) was used at the time of the seeding, followed by culture until about 95% confluency was achieved.

The iPS cells cultured in the 12-well plate were first cultured for 1 day in RPMI 1640 medium (Sigma-Aldrich Co., LLC) containing 1 $\mu$M CHIR99021 (FUJIFILM Wako Pure Chemical Corporation), 100 ng/mL Activin A (R&D Systems Inc.), P/S (Penicillin-Streptomycin Solution ($\times$100), FUJIFILM Wako Pure Chemical Corporation), 2 mM Gln (2 mM L-glutamine, FUJIFILM Wako Pure Chemical Corporation), and B27 (0.5$\times$ B27 Supplement Minus Vitamin A, Thermo Fisher Scientific Inc.), and then cultured for 2 days in RPMI 1640 medium (Sigma-Aldrich Co. LLC) containing 100 ng/mL Activin A (R&D Systems Inc.), P/S (FUJIFILM Wako Pure Chemical Corporation), 2 mM Gln (FUJIFILM Wako Pure Chemical Corporation), and B27 (Thermo Fisher Scientific Inc.) to be induced to differentiate into definitive endoderm cells.

(Reference Example 2) Induction of Differentiation from Definitive Endoderm Cells into Intestinal Progenitor Cells

This Reference Example describes a basal medium for inducing differentiation from definitive endoderm cells into intestinal progenitor cells.

To induce differentiation from definitive endoderm cells into intestinal progenitor cells, DMEM (DMEM-High Glucose medium, FUJIFILM Wako Pure Chemical Corporation) medium containing 10% KSR (10% knockout serum replacement, Thermo Fisher Scientific Inc.), 1% NEAA (1% non-essential amino acid solution, Thermo Fisher Scientific Inc.), P/S (FUJIFILM Wako Pure Chemical Corporation), 1 mM Gln (FUJIFILM Wako Pure Chemical Corporation), and B27 (Thermo Fisher Scientific Inc.) was used as medium B. In Examples described below, various additives were added to the medium B to seek to optimize culture conditions.

(Reference Example 3) Induction of Differentiation from Intestinal Progenitor Cells into Enterocyte-like Cells (ELCs)

This Reference Example describes a basal medium for inducing differentiation from intestinal progenitor cells into enterocyte-like cells (ELCs). In a passage culture operation in cell culture, culture was performed under a condition containing 10 μM Y-27632 (FUJIFILM Wako Pure Chemical Corporation), which was a ROCK inhibitor, for 2 days after passage.

To induce differentiation from intestinal progenitor cells into enterocyte-like cells (ELCs), DMEM-High Glucose medium containing 50 ng/mL EGF (R&D Systems Inc.), 2 μM SB431542 (FUJIFILM Wako Pure Chemical Corporation), 100 nM VD3 (1α,25-Dihydroxyvitamin D3, Cayman Chemical Company), 3 nM LY2090314, 10% KSR (Thermo Fisher Scientific Inc.), 1% NEAA (Thermo Fisher Scientific Inc.), P/S (FUJIFILM Wako Pure Chemical Corporation), 1 mM Gln (FUJIFILM Wako Pure Chemical Corporation), and B27 (Thermo Fisher Scientific Inc.) was used as medium C. In Examples described below, various additives were added to the medium C to seek to optimize culture conditions.

(Example 1) Investigation of Method of Inducing Differentiation from Definitive Endoderm Cells into Intestinal Progenitor Cells (Screening 1)

This Example examined a differentiation-inducing action in a culture system containing each concentration of retinoic acid or a GSK3β inhibitor, which was known to be important in differentiation from definitive endoderm. To induce differentiation from definitive endoderm cells into intestinal progenitor cells, the medium B shown in Reference Example 2 was used as a basal medium. A differentiation-inducing action was examined in a culture system supplemented with 3 μM, 10 μM, 20 μM, or 30 μM of all-trans-Retinoic Acid (FUJIFILM Wako Pure Chemical Corporation) as retinoic acid, or 3 nM, 10 nM, 20 nM, or 30 nM of LY2090314 (MedChemExpress LLC) or 3 μM, 10 μM, or 20 μM of CHIR99021 (FUJIFILM Wako Pure Chemical Corporation) as a GSK3β inhibitor. In this Example and Examples described below, retinoic acid is sometimes abbreviated as RA or R, LY2090314 is sometimes abbreviated as LY or L, and CHIR99021 is sometimes abbreviated as CHIR or C.

To induce differentiation from definitive endoderm cells into intestinal progenitor cells, a system containing each concentration of RA, LY, or CHIR in the medium B was allowed to act for 4 days. Then, RNAs were recovered and analyzed for gene expression levels of an intestinal progenitor cell marker CDX2, a hepatic progenitor cell marker AFP, and a pancreatic progenitor cell marker PDX1 by quantitative RT-PCR (qRT-PCR). The quantitative RT-PCR was performed according to a common method. Total RNAs were recovered and cDNAs were synthesized. Complementary DNAs (cDNAs) were subjected to qRT-PCR with Fast SYBR Green Master Mix (Applied Biosystems) and quantification with StepOnePlus Real time PCR system (Applied Biosystems). A glyceraldehyde 3-phosphate dehydrogenase (GAPDH) gene was used as an internal standard gene. In addition, each gene expression level of DMSO-treated groups, which were controls, was set to 1.0. Data was shown with n=3, mean ± standard deviation, but n=2 for each LY (10 nM and 30 nM). A detection limit was represented by N.D.

The results demonstrated that the system containing RA exhibited high expression levels of CDX2 and PDX1 at each concentration, suggesting differentiation directed to the intestine or the pancreas. The system containing LY exhibited the highest expression of CDX2 and low expressions of AFP and PDX1 at 20 nM, suggesting intestine-specific differentiation. The system containing CHIR exhibited the highest expression of CDX2 and low expressions of AFP and PDX1 at 10 μM, similarly suggesting intestine-specific differentiation (FIG. 1).

(Example 2) Investigation of Method of Inducing Differentiation from Definitive Endoderm Cells into Enterocyte-like Cells (ELCs) (Screening 2)

This Example investigated a method of inducing differentiation in a step of inducing differentiation from definitive endoderm cells into intestinal progenitor cells in a step of inducing differentiation into enterocyte-like cells (ELCs). To induce differentiation from definitive endoderm cells into intestinal progenitor cells, the medium B shown in Reference Example 2 was used as a basal medium. A system containing each combination of 10 μM retinoic acid, 20 nM LY2090314, and 10 μM CHIR99021 in the medium B in a step of introducing differentiation from definitive endoderm cells into intestinal progenitor cells on days 3 to 4 (day 3-4) and days 5 to 6 (day 5-6) after the start of the induction of differentiation was allowed to act for 4 days according to the protocol illustrated in FIG. 2(1) and each of the combination conditions shown in Table 3. In this Example, retinoic acid, LY2090314, and CHIR99021 are abbreviated as R, L, and C, respectively (Table 3 and FIG. 2(2)).

Table 3

| | day 3-4 | day 5-6 |
|---|---|---|
| 2 | L | L |
| 3 | L | C |
| 15 | C | L+R+C |
| 27 | L+C | R+L |
| 29 | L+C | L+R+C |
| 40 | R+C | L+C |
| 42 | R+C | R+C |
| 49 | L+R+C | R+C |
| 50 | L+R+C | L+R+C |

After the induction of differentiation into the intestinal progenitor cells, the intestinal progenitor cells were cultured for 20 days in the medium C shown in Reference Example 3 according to the protocol illustrated in FIG. 2(1) in order to induce further differentiation into enterocyte-like cells (ELCs). On days 11 to 14 (day 11-14) after the start of the induction of differentiation, 10 nM vinblastine (FUJIFILM Wako Pure Chemical Corporation) was also allowed to act.

After the induction of differentiation into the enterocyte-like cells (ELCs), RNAs were recovered and analyzed for gene expression levels of a enterocyte marker VIL1, a major drug metabolic enzyme CYP3A4, and a major drug efflux transporter MDR1 by qRT-PCR. The quantitative RT-PCR was performed by the same procedure as in Example 1. Each gene expression level in an adult small intestine was set to 1.0. Data was shown with n=3, mean $\pm$ standard deviation.

The results revealed that in the step of inducing differentiation from definitive endoderm cells into intestinal progenitor cells, the culture in the system containing CHIR99021 (10 $\mu$M) (each of Conditions 15, 27, 29, 40, 42, 49, and 50 shown in Table 3) provided high expressions of VIL1, CYP3A4, and MDR1, in particular, a high expression of CYP3A4 (FIG. 2(2)). In particular, it was found that the system (49) in which the cells were cultured in the system containing L, C, and R on days 3 to 4 (day 3-4) after the start of the induction of differentiation, and containing R and C on days 5 to 6 (day 5-6) after the start of the induction of differentiation provided high expressions of VIL1, CYP3A4, and MDR1 (FIG. 2(2)).

(Example 3) Investigation of Method of Inducing Differentiation from Intestinal Progenitor Cells into Enterocyte-like Cells (ELCs)

This Example investigated the action of a MEK inhibitor in a stage of inducing differentiation from intestinal progenitor cells into enterocyte-like cells (ELCs). PD0325901 (MedChemExpress LLC) was used as the MEK inhibitor. PD0325901 is sometimes abbreviated as PD in this Example and Examples described below. In a step from definitive endoderm cells to intestinal progenitor cells, culture was performed under Conditions 2 and 3 shown in Table 3 in Example 2 according to the protocol illustrated in FIG. 3(1). The conditions are specifically as described below.

- Condition 2: LY→LY (day 3-4: LY (20 nM), day 5-6: LY (20 nM))
- Condition 3: LY→CHIR (day 3-4: LY (20 nM), day 5-6: CHIR (10 $\mu$M))

The intestinal progenitor cells produced under each of the conditions were cultured in the medium C shown in Reference Example 3 for 10 days, then passaged, and further cultured in a PD(+) system containing PD (3 $\mu$M) in the medium C and a PD(-) system containing no PD in the medium C for 10 days. Thus, the cells were induced to differentiate into enterocyte-like cells (ELCs). Subsequently, RNAs were recovered by the same procedure as in Example 1, and analyzed for each gene expression level of VIL1, CYP3A4, and MDR1 by qRT-PCR by the same method as in Example 2. Each gene expression level in an adult small intestine was set to 1.0. Data was shown with n=3, mean $\pm$ standard deviation. The results demonstrated that PD(+) exhibited clearly higher values for the major drug metabolic enzyme CYP3A4 and the major drug efflux transporter MDR1 (FIG. 3(2)).

Further, CYP3A4 activity was checked. The CYP3A4 activity was measured with P450-GloTM CYP3A4 Assay Kits (Promega Corporation). Luminescence was measured with a luminometer (Lumat LB 9507, Berthold Technologies GmbH & Co. KG) through use of Luciferin-IPA as a substrate for CYP3A4. The CYP3A4 activity was corrected by the amount of a protein per well measured with BCA Protein Assay Kit (Thermo Fisher Scientific Inc.). The results demonstrated that PD(+) also provided a clearly higher value of the CYP3A4 activity (FIG. 3(3)).

(Example 4) Investigation of Optimized Method of Inducing Differentiation from Intestinal Progenitor Cells into Enterocyte-like Cells (ELCs)

This Example investigated an optimized method of inducing differentiation into enterocyte-like cells (ELCs). In a step from definitive endoderm cells to intestinal progenitor cells, culture was performed under Conditions 2, 3, and 49 shown in Table 3 in Example 2 according to the protocol illustrated in FIG. 4(1). The conditions are specifically as described below.

- Condition 2: LY→LY (day 3-4: LY (20 nM), day 5-6: LY (20 nM))
- Condition 3: LY→CHIR (day 3-4: LY (20 nM), day 5-6: CHIR (10 $\mu$M))
- Condition 49: L+R+C→R+C (day 3-4: LY (20 nM)+RA (10 $\mu$M)+CHIR (10 $\mu$M), day 5-6: +RA (10 $\mu$M)+CHIR (10 $\mu$M)

The intestinal progenitor cells produced under each of the conditions were cultured in the medium C shown in Reference Example 3 for 10 days, then passaged, and further cultured in a PD(+) system containing PD (3 $\mu$M) in the medium C for 10 days. Thus, the cells were induced to differentiate into enterocyte-like cells (ELCs). Subsequently, RNAs were recovered by the same procedure as in Example 1, and analyzed for each gene expression level of VIL1, CYP3A4, and MDR1 by qRT-PCR by the same method as in Example 2. Each gene expression level in an adult small intestine was set to 1.0. Data was shown with n=3, mean $\pm$ standard deviation. The results demonstrated that Condition 49 (L+R+C→R+C) provided the highest value of each gene expression level of each of VIL1, CYP3A4, and MDR1 (FIG. 4(2)) and also provided a high value of CYP3A4 activity (FIG. 4(3)).

(Example 5) Organoids and Monolayers Derived from Enterocyte-like Cells (ELCs)

In this Example, organoids and a monolayer produced from enterocyte-like cells (ELCs) were analyzed for gene expression levels of pharmacokinetics-related molecules and intestinal differentiation markers by qRT-PCR. A protocol for producing organoids and a monolayer from enterocyte-like cells (ELCs) is shown in FIG. 5(1).

(1) Procedure for Inducing Differentiation from Human iPS into Enterocyte-like Cells (ELCs)

[0064]

　(a) Induction of Differentiation from Human iPS Cells into Definitive Endoderm Cells
　Induction of differentiation into definitive endoderm cells was performed by the method described in Reference Example 1.
　(b) Induction of Differentiation from the Definitive Endoderm Cells into Intestinal Progenitor Cells
　Culture was performed in a culture system shown in Condition 49 in Example 2 to induce differentiation into intestinal progenitor cells.
　(c) Induction of Differentiation from the Intestinal Progenitor Cells into Enterocyte-like Cells (ELCs)

Culture was performed in the medium C without PD shown in Example 3 or 4 for 10 days from days 7 to 17 (days 7-17) after the start of the induction of differentiation. On day 17 (day 17) after the start of the induction of differentiation, the cells were detached with TrypLE Select Enzyme (Thermo Fisher Scientific Inc.), and 2.5$\times$10$^6$ cells/mL of the cells were seeded and passaged in a 96-well plate for cell culture (Thermo Fisher Scientific Inc.) or a cell culture insert (24-well plate, 0.4 $\mu$M pore size, PET membrane, Corning Incorporated) coated with Matrigel, and cultured for 10 days with the medium K shown in Table 1 described above to induce differentiation into enterocyte-like cells (ELCs) (day 27).

(2) Production of Organoids Derived from Enterocyte-like Cells (ELCs)

The enterocyte-like cells (ELCs) produced through culture until day 17 (day 17) or day 27 (day 27) after the start of the induction of differentiation were detached with TrypLE Select Enzyme (Thermo Fisher Scientific Inc.), and suspended in Matrigel, and 1.0$\times$10$^6$ cells/mL of the cells were seeded by from 40 $\mu$L to 60 $\mu$L in each well of a 24-well plate (Thermo Fisher Scientific Inc.) and cultured in the medium F shown in Table 2 described above. Medium change was performed every 2 days. Thus, organoids were produced. The organoids produced in this Example are hereinafter referred to as "ELC organoids."

The ELC organoids thus produced were subjected to maintenance culture in the medium F. The passage interval was set to about 1 week. The ELC organoids were suspended together with Matrigel in ice-cold advanced DMEM-F12 (Gibco) and centrifuged twice. After that, the supernatant was discarded, and the cells were recovered, resuspended in fresh Matrigel, seeded by from 40 $\mu$L to 60 $\mu$L in the center of each well of a 24-well plate, and cultured in the medium F.

Subsequently, medium change was performed every 2 to 3 days. The sample cultured in this state is represented as "3D" (FIG. 5(1)).

(3) Production of ELC Monolayer Film from ELC Organoids

Advanced DMEM/F-12, to which Matrigel had been added at 2 vol%, was loaded into each well of a cell culture insert (24-well plate, 0.4 $\mu$M pore size, PET membrane, Corning Incorporated) or a 96-well plate (Thermo Fisher Scientific Inc.), and incubation was performed at 37°C for 1 hour to coat the culture tool with Matrigel. Next, the ELC organoids produced and subjected to maintenance culture in the section (2) were washed together with Matrigel with 0.5 mM EDTA/PBS, and the ELC organoids were suspended in TrypLE Select Enzyme, incubated at 37°C for 10 minutes, and then dissociated into single cells by 10 to 20 times of vigorous pipetting. The cells were recovered by centrifugation and resuspended in the medium K (FIG. 5(1)). The cells were passed through a 70 $\mu$m cell strainer (Corning Incorporated) and then seeded at a cell density of from $2.5\times10^6$ cells/mL to $1.0\times10^6$ cells/mL in a 24-well cell culture insert (Corning Incorporated) coated with Matrigel as a base material for two-dimensional culture. Medium change was performed every 1 to 2 days with the medium K without 10 $\mu$M Y-27632, and the passaged cells were seeded and then cultured for from 3 days to 7 days to produce a monolayer. The organoid monolayers produced in this Example is hereinafter referred to as "ELC monolayers." In culture in the medium F, coating was performed with iMatrix-511 solution (Nippi Incorporated) in the same manner, and medium change after seeding was performed with the medium F. The sample cultured under such state is represented as "2D" (FIG. 5(1)).

In this Example, the medium K was added to only the apical membrane side (apical side: A) of the cell culture insert, followed by culture. The apical membrane side (apical side: A) is hereinafter sometimes simply referred to as "upper stage A" or "A". Likewise, a basolateral membrane side (basolateral side: B) is hereinafter sometimes simply referred to as "lower stage B" or "B".

(4) Expressions of Various Genes in ELC Organoids and ELC Monolayers

Various analyses were performed under a state in which the ELC organoids produced and subjected to maintenance culture in the section (2) were passaged and then cultured for 7 days (3D), and under a state in which the monolayers produced in the section (3) were cultured for 7 days (2D). Respective gene expression levels of pharmacokinetics-related molecules (MDR1, BCRP, PEPT1, CYP3A4, UGT1A1, CES1, and CES2) and intestinal differentiation markers (CDX2, VIL1, CHGA, MUC2, LYZ, LGR5, and EPCAM) were analyzed by qRT-PCR. All data represent mean $\pm$ S.D. (n=3, biological replicate). A gene expression level of GAPDH was set to 1 (FIG. 5(2) and FIG. 5(3)).

The results of qRT-PCR analysis showed that 2D groups had increased gene expression levels of many genes regardless of when organoids were produced. In addition, under both 3D and 2D conditions, the organoids formed from the cells on day 27 exhibited gene expression levels of many genes equal to or more than those of the organoids formed from the cells on day 17. In particular, day 27 2D groups exhibited gene expression levels near those of a human adult small intestine in each of the intestinal differentiation marker CDX2, which was used as an indicator of intestinal differentiation, and the drug metabolic enzyme CYP3A4, which was highly expressed in the small intestine. The results suggested that it was appropriate to use enterocyte-like cells (ELCs) passed through the induction of differentiation to day 27 in order to produce organoids with high functionality, and that the organoids thus produced had more improved functions when formed into monolayers.

(Example 6) Evaluation of ELC Monolayers

In this Example, CYP3A4 activity, P-gp activity, and CES2 activity were measured for the ELC monolayers (2D) produced in Example 5(3).

The CYP3A4 activity was measured by the method shown in Example 3.

P-gp is an efflux transporter, and its activity was measured by allowing 1 $\mu$M digoxin to act through use of digoxin, $[^3H(G)]$- (PerkinElmer) and digoxin (Asahi Kasei Corporation) as substrate compounds, and allowing 100 $\mu$M verapamil to act as an inhibitor. Papp and ER were calculated with the following formula:

$$\mathrm{Papp} = \delta C_r / \delta_t \times V_r / (S \times C_0)$$

$\delta C_r/\delta_t$: (when temporal sampling was performed) concentration change rate (mM/sec) on the receiver side
$\delta C_r$: (when no temporal sampling was performed) final concentration (mM) on the receiver side
$\delta_t$: (when no temporal sampling was performed) incubation time (sec)

$V_r$: volume (mL) on the receiver side

S: area (cm$^2$) of a cell culture insert

$C_0$: initial concentration (mM) on the donor side

An efflux ratio (ER) was calculated with the following formula. ER=Papp B to A/Papp A to B

$P_{app\ A\ to\ B}$ represents a Papp value in a direction from the apical membrane side (apical side: A) to the basolateral membrane side (basolateral side: B).

$P_{app\ B\ to\ A}$ represents a Papp value in a direction from the basolateral membrane side (B) to the apical membrane side (A).

Measurement of the CES2 activity was performed with fluorescein diacetate, which was a substrate compound for CES2. First, distilled water containing 50 mM Tris-HCl (pH 7.4), 150 mM NaCl (FUJIFILM Wako Pure Chemical Corporation), 0.5 vol% Triton X-100, and 1 mM EDTA was added to the cells, followed by incubation on ice for 20 minutes, and a lysate was recovered. The lysate was centrifuged at 4°C and 15,000 g for 15 minutes, and the supernatant was recovered as S9 fraction. Next, the S9 fraction was diluted with distilled water to 20 $\mu$g protein/mL, followed by addition of fluorescein diacetate (FUJIFILM Wako Pure Chemical Corporation) thereto so as to provide a final concentration of 10 $\mu$M. The mixture was incubated at 37°C for 15 minutes and then mixed with an equal amount of ice-cold acetonitrile (FUJIFILM Wako Pure Chemical Corporation) to quench the reaction. Finally, the resultant was centrifuged, and the supernatant was measured for fluorescent intensity (excitation wavelength: 485 nm, fluorescence wavelength: 535 nm) with a multimode microplate reader TriStar LB941 (Berthold Technologies GmbH & Co. KG). The amount of fluorescence (fluorescein) generated in the reaction liquid was determined from the fluorescent intensity thus obtained and a calibration curve, and defined as an activity value. Further, 1 mM loperamide (FUJIFILM Wako Pure Chemical Corporation) was added as a CES2 inhibitor, and preincubation was performed at 37°C for 5 minutes before the addition of the substrate compound.

The results are shown in FIG. 6. All data represent mean $\pm$ S.D. (n=3, biological replicate). A data label of the P-gp activity represents an efflux ratio (ER) value. In each activity measurement, the organoids formed from the cells on day 27 (3D) exhibited higher activity. This suggested that just like Example 5, it was appropriate to use enterocyte-like cells (ELCs) passed through the induction of differentiation to day 27 in order to produce organoids with high functionality.

(Example 7) Evaluation of ELC Organoids and ELC Monolayers

This Example investigated ELC organoids, and an ELC monolayer cultured in the medium K or the medium F. The ELC organoids produced in Example 5(2) (day 27 3D) were subjected to maintenance culture and then dissociated into single cells, and the cells were cultured as a monolayer for 6 days by the same procedure as in Example 5(3) except for being cultured as a monolayer with the medium K for 1 day, followed by medium change into the medium K or the medium F. Thus, an ELC monolayer was produced (FIG. 7(1)). The ELC monolayer produced with the medium K was defined as day 27 2D K, and the ELC monolayer produced with the medium F was defined as day 27 2D F. Each sample was analyzed for gene expression levels of pharmacokinetics-related molecules (MDR1, BCRP, PEPT1, CYP3A4, UGT1A1, CES1, and CES2) and intestinal differentiation markers (CDX2, VIL1, CHGA, MUC2, LYZ, LGR5, and EPCAM) by qRT-PCR as in Example 5. Further, CYP3A4 activity was also measured by the method shown in Example 3.

The data shown in FIG. 7(2) and FIG. 7(3) represent mean $\pm$ S.D. (n=3, biological replicate). A gene expression level of GAPDH was set to 1. The data shown in FIG. 7(4) was CYP3A4 activity of day 27 2D K or day 27 2D F, with all data representing mean $\pm$ S.D. (n=3, biological replicate). In qRT-PCR analysis, a day 27 2D K group exhibited higher gene expression levels in many genes including CYP3A4 relative to a day 27 2D F group. Similar results were obtained for CYP3A4 activity. These suggested that in monolayer culture, use of the medium K resulted in higher functionality.

(Example 8) Investigation of Culture Period and Culture Conditions of ELC Monolayer

This Example investigated a culture period and culture conditions of an ELC monolayer. The ELC monolayer produced in Example 7 (day 27 2D K) was measured for a transepithelial electrical resistance value (trans endothelial electrical resistance; TEER), CYP3A4 activity, and P-gp activity in change of a culture period in use of the medium K (FIG. 8(1)). Further, a cell culture insert (Corning Incorporated) used in monolayer culture was examined for culture with addition of the medium to only an upper stage A and for culture with addition of the medium to the upper stage A and a lower stage B (A&B group). The TEER was calculated according to the following formula:

TEER ($\Omega \cdot$cm$^2$)={(actually measured value of sample)-(actually measured value of polycarbonate membrane (blank))}x (surface area of membrane)

In addition, a rate of cells positive for EpCAM, which was an intestinal epithelial cell marker, was calculated by flow cytometry, for each of the ELC organoids and the ELC monolayers that experienced different culture periods between addition of the medium K to the upper stage A and to the lower stage B in the cell culture insert.

The results were shown in FIG. 8(2) to FIG. 8(4). Day 3, day 5 and day 7 shown in FIG. 8 represent the cumulative numbers of culture days in the medium K. All data represent mean ± S.D. (n=3, biological replicate). A data label in FIG. 8(4) represents an efflux ratio (ER). The results in FIG. 8(2) to FIG. 8(4) revealed no adverse influence caused by culture with further addition of the medium to the lower stage B. The CYP3A4 activity was most highly exerted on day 5. With regard to the P-gp activity, the A&B group exhibited high efflux ratios, with a particularly high value on day 3. These suggested that for production of enterocyte-like cells (ELCs) with high functionality, it is appropriate to perform culture with addition of the medium to the upper stage A and the lower stage B in the cell culture insert in monolayer culture, for a cumulative culture period of 3 days in the medium K in view of transporter activity, or for a cumulative culture period of 5 days in the medium K in view of CYP3A4 activity.

In addition, the results in FIG. 8(5) demonstrated that both of the ELC organoids and each culture period of the ELC monolayer cultured with addition of the medium to the upper stage A and the lower stage B in the cell culture insert exhibited 100% in a rate of cells positive for EpCAM, which was an intestinal epithelial cell marker. This suggested that the ELC organoids and the ELC monolayer were formed only of intestinal epithelial cells.

(Example 9) Influence 1 of Humoral Factor in ELC Monolayer Culture

This Example investigated an influence of a humoral factor in ELC monolayer culture. The ELC monolayer produced in Example 7 (day 27 2D K) was cultured for 1 day in the medium K, then further cultured for 6 days in the medium K with elimination of a particular humoral factor selected from EGF, SB431542, LY2090314, Vitamin D3, and PD0325901, and measured for CYP3A4 activity and P-gp activity. In this Example, culture was performed with addition of the medium to only an upper stage A in a cell culture insert (Corning Incorporated) used in monolayer culture (FIG. 9(1)).

The results were shown in FIG. 9(2) and FIG. 9(3). All data represent mean ± S.D. (n=3, biological replicate). A data label showing the P-gp activity represents an efflux ratio (ER) value. The CYP3A4 activity was significantly increased by eliminating EGF, SB431542, or the like, but greatly decreased upon elimination of Vitamin D3 or PD0325901. In particular, the elimination of EGF had no influence on the P-gp activity. These suggested that modification of the medium K allowed production of enterocyte-like cells (ELCs) with higher functionality.

(Example 10) Influence 2 of Humoral Factor in ELC Monolayer Culture

This Example investigated an influence of a humoral factor in ELC monolayer culture. The ELC monolayer produced in Example 7 (day 27 2D K) was cultured for 1 day in the medium K, then further cultured for 6 days in the medium K with elimination of EGF and Vitamin D3, and measured for CYP3A4 activity, TEER, and P-gp activity. In this Example, culture was performed with addition of the medium to only an upper stage A in a cell culture insert (Corning Incorporated) used in monolayer culture (FIG. 10 (1)).

The results were shown in FIG. 10(2) to FIG. 10(4). All data represent mean ± S.D. (n=3, biological replicate). A data label showing the P-gp activity represents an efflux ratio (ER). Effects of elimination of EGF and Vitamin D3 acted antagonistically, and thus even elimination of both had no adverse influence on the CYP3A4 activity or the TEER. In terms of the P-gp activity, elimination of a humoral factor reduced an efflux ratio, but had no adverse influence on migration from a lower stage B to an upper stage A (B to A), which was actual efflux activity. These suggested that elimination of EGF and Vitamin D3 were able to improve the medium K. Vitamin D3 is used as a CYP3A4 inducer, and hence this modification allows establishment of a culture system that is also applicable to an induction test.

(Example 11) Influence 3 of Humoral Factor in ELC Monolayer Culture

This Example investigated induction of CYP3A4 in an ELC monolayer. The ELC monolayer produced in Example 7 (day 27 2D K) was cultured for 1 day in the medium K, and then further cultured for 2 days in the medium K (VD3-) with elimination of Vitamin D3, a CYP3A4 inducer, or in normal medium K (K) (FIG. 11(1)). At that time, CYP3A4 activity was measured for each sample obtained when 20 μM rifampicin (RIF, FUJIFILM Wako Pure Chemical Corporation), which was a CYP3A4 inducer, was not allowed to act on each medium (RIF-) or was allowed to act on the medium for 48 hours (RIF+). Further, a gene expression level of CYP3A4 was analyzed by qRT-PCR. In this Example, a 96-well plate for cell culture (Thermo Fisher Scientific Inc.) was used in monolayer culture.

The results were shown in FIG. 11(2) and FIG. 11(3). All data represent mean ± S.D. (n=3, biological replicate). CYP3A4 activity and a gene expression level of RIF- in each condition were set to 1. The numerical values on the graphs represent induction rates. The results in FIG. 11(2) and FIG. 11(3) showed that the action of RIF increased activity and a gene expression level of CYP3A4 regardless of the presence and absence of Vitamin D3 in the medium. In addition, the elimination of Vitamin D3 exhibited a larger rise in gene expression level of CYP3A4. These suggested that the enterocyte-like cells (ELCs) of the present invention had an ability to induce CYP3A4, and thus demonstrated that the cells were also applicable to an induction test of CYP3A4.

(Example 12) Transport Test with RI-labeled Compound for ELC Monolayer

This example examined compound transportation ability of an ELC monolayer. A transportation test was performed for the ELC monolayer produced in Example 7 (day 27 2D K). In the transportation test, migration of a RI-labeled compound from an upper stage A to a lower stage B or from a lower stage B to an upper stage A in a cell culture insert (Corning Incorporated) was examined by P-gp activity and BCRP activity.

Culture was performed according to the protocol illustrated in FIG. 12(1). An organoid monolayer on a cell culture insert (Corning Incorporated) was washed with PBS. After washing, HBSS solution was added in a volume of 250 $\mu$L on the upper stage A and in a volume of 1 mL on the lower stage B in the cell culture insert, followed by pre-incubation at 37°C for 30 minutes. After the pre-incubation, a RI-labeled compound was added to the upper stage A or the lower stage B (donor side), and HBSS with no substrate compound was added to a compartment on the opposite side (receiver side). After action for 90 minutes, 200 $\mu$L of a sample was taken from the receiver side, and its radiation dose was quantified with MicroBeta2 (PerkinElmer). In the pre-incubation and the transport test, an inhibitor or its solvent DMSO (0.1%) was allowed to act on the upper stage A.

The P-gp activity was measured in the same manner as the method shown in Example 6. The BCRP activity was measured by using estrone 3-sulfate [6,7-$^3$H(N)] ammonium salt (American Radiolabeled Chemicals, Inc.) and estrone sulfate to be allowed to act as 10 $\mu$M estrone sulfate, and using 100 $\mu$M Ko143 as an inhibitor.

Calculation of Papp and ER

Papp was calculated with the following formula.

$$\mathrm{Papp} = \delta C_r / \delta_t \times V_r / (S \times C_0)$$

$\delta C_r/\delta_t$: (when temporal sampling was performed) concentration change rate (mM/sec) on the receiver side
$\delta C_r$: (when no temporal sampling was performed) final concentration (mM) on the receiver side
$\delta_t$: (when no temporal sampling was performed) incubation time (sec)
$V_r$: volume (mL) on the receiver side
S: area (cm$^2$) of a cell culture insert
$C_0$: initial concentration (mM) on the donor side

An efflux ratio (ER) was calculated with the following formula.

ER=Papp B to A/Papp A to B
Papp A to B: Papp in a direction from the apical membrane side (A) to the basement membrane side (B)
Papp B to A: Papp in a direction from the basement membrane side (B) to the apical membrane side (A)

FIG. 12(2) shows P-gp transport activity in a state of culturing ELC organoids as a monolayer, and FIG. 12(3) shows results of measuring BCRP transport activity. All data represent mean $\pm$ S.D. (n=3, biological replicate). The numerical values on the graphs represent ER values. The cells were confirmed to have both P-gp transport activity and BCRP transport activity.

(Example 13) Influence of Cryopreservation on Function of ELC Monolayer

This example examined influence of cryopreservation on a function of an ELC monolayer. Culture was performed according to a protocol illustrated in FIG. 13(1). Expression of each gene was examined for an ELC monolayer derived by culturing the ELC organoids produced in Example 5(2) (day 27 3D), as a monolayer with use of the medium K for 3 days (M-ELC mono), and an ELC monolayer derived by dissociating the ELC organoids into single cells, cryopreserving the cells, then thawing and culturing the cells as a monolayer with use of medium K for 3 days (F-ELC mono). Each of the ELC monolayers was measured for gene expression of each of pharmacokinetics-related molecules and intestinal differentiation markers. Gene expression levels were measured by qRT-PCR.

CYP3A4 activity was measured by the method shown in Example 3. The ELC monolayers were cultured with the medium K for 7 days.

P-gp activity was measured in the same manner as the method shown in Example 6. The ELC monolayers were cultured with the medium K for 3 days. As a substrate compound, 10 μM Rho123 (Sigma-Aldrich Co., LLC) was allowed to act.

The results of analyzing gene expression levels were shown in FIG. 13(2). All data represent mean ± S.D. (n=3, biological replicate). A gene expression level of GAPDH was set to 1. FIG. 13(3) shows CYP3A4 activity, and FIG. 13(4) shows P-gp activity. The numerical value on the graph in FIG. 13(4) represents an efflux level (ER value). The results in FIG. 13(2) to FIG. 13(4) revealed that there was also no significant influence on a cell function as enterocyte-like cells (ELCs) in an ELC monolayer produced from cryopreserved ELC organoids.

(Example 14) Influence of Long-term Culture of ELC Organoids on Function of ELC Monolayer

This Example investigated an influence of long-term culture of an ELC organoid on a function of an ELC monolayer. ELC organoids through various passages were examined for expression of each gene in the ELC monolayer produced as in Example 7. Each of the ELC monolayer was measured for expression of each gene of pharmacokinetics-related molecules and intestinal differentiation markers. Gene expression levels were measured by qRT-PCR.

CYP3A4 activity was measured by the method shown in Example 3.

P-gp activity was measured in the same manner as the method shown in Example 6. As a substrate compound, 10 μM Rho123 (Sigma-Aldrich Co., LLC) was acted.

The results were shown in FIG. 14(1) to FIG. 14(3). The numeral following P shown in FIG. 14(1) represents the number of passages of ELC organoids used for producing an ELC monolayer. All data represent mean ± S.D. (n=3, biological replicate). FIG. 14(2) shows CYP3A4 activity of a monolayer derived by using ELC organoids with a passage number of 51, and FIG. 14(3) shows P-gp activity of a monolayer derived by using ELC organoids with a passage number of 52. The numerical values on the graph in FIG. 14(3) represent efflux levels (ER values). The results in FIG. 14(1) to FIG. 14(3) revealed that there was also no significant influence on a cell function as enterocyte-like cells (ELCs) in an ELC monolayer produced from ELC organoids that experienced long-term culture.

Industrial Applicability

The enterocyte-like cells of the present invention maintain high activities of drug metabolic enzymes as well as gene expressions of the drug metabolic enzymes. Thus, the enterocyte-like cells can be effectively utilized for in vitro pharmacokinetic evaluation. The enterocyte-like cells of the present invention are also applicable to an induction test of CYP3A4. In addition, enterocyte-like cells produced by the method of the present invention and a cell population thereof can be cryopreserved and passaged, can provide a large amount of cells with excellent performance in the same lot, and can semipermanently supply enterocyte-like cells with constant quality.

Such enterocyte-like cells and a cell population thereof with an excellent quality can be used in pharmacokinetic evaluation and drug toxicity evaluation kits. It has hitherto been difficult to perform in vitro pharmacokinetic evaluation, a drug toxicity test in enterocyte-like cells, and the like under certain criteria, but a safety evaluation system with large scale and high accuracy can be provided, offering great benefit for increasing success rates of research and development, and reducing cost and time.

**Claims**

1. A method of inducing differentiation from pluripotent stem cells into enterocyte-like cells, the method comprising the following steps (1) and (2):

   (1) a step of inducing differentiation from pluripotent stem cells into definitive endoderm cells; and
   (2) a step of culturing the definitive endoderm cells in a system containing CHIR99021, followed by induction of differentiation into intestinal progenitor cells.

2. The method of inducing differentiation according to claim **1,** wherein the step (2) includes the following step (2a) and step (2b):

   (2a) a step of culturing the definitive endoderm cells in a system containing CHIR99021 and further containing one or more kinds selected from LY2090314 and retinoic acid; and
   (2b) a step of further culturing the definitive endoderm cells in a system containing two or more kinds selected from CHIR99021, retinoic acid, and LY2090314.

3. The method of inducing differentiation into enterocyte-like cells according to claim 2, wherein the step (2a) is performed in a system containing CHIR99021 and retinoic acid, and the step (2b) is performed in a system containing CHIR99021 and further containing retinoic acid and/or LY2090314.

4. The method of inducing differentiation into enterocyte-like cells according to claim 2, wherein the step (2a) is performed in a system containing LY2090314 and CHIR99021, and the step (2b) is performed in a system containing CHIR99021 and retinoic acid.

5. The method of inducing differentiation into enterocyte-like cells according to claim 2, wherein the step (2a) is performed in a system containing LY2090314, CHIR99021, and retinoic acid, and the step (2b) is performed in a system containing CHIR99021 and retinoic acid.

6. A method of inducing differentiation from pluripotent stem cells into enterocyte-like cells, the method comprising the following steps (1) to (3):

(1) a step of introducing differentiation from pluripotent stem cells into definitive endoderm cells;
(2') a step of culturing the definitive endoderm cells in a system containing LY2090314 and/or CHIR99021, followed by induction of differentiation into intestinal progenitor cells; and
(3) a step of culturing the intestinal progenitor cells in a system containing LY2090314 and/or a MEK inhibitor.

7. The method of introducing differentiation according to claim 6, wherein the step (2') includes the following step (2'a) and step (2'b):

(2'a) a step of culturing the definitive endoderm cells in a system containing LY2090314 for 2 days; and
(2'b) a step of culturing the definitive endoderm cells in a system containing LY2090314 or CHIR99021 for 2 days.

8. The method of inducing differentiation according to claim 6, wherein the step (3) includes the following step (3a) and step (3b):

(3a) a step of culturing the intestinal progenitor cells in a system containing LY2090314 for 10 days; and
(3b) a step of culturing the intestinal progenitor cells in a system containing LY2090314 and a MEK inhibitor for 10 days.

9. The method of inducing differentiation according to claim 6 or 8, wherein the MEK inhibitor is PD0325901.

10. A method of producing a cell population of enterocyte-like cells derived from pluripotent stem cells, the method comprising the following steps (I) to (IV):

(I) a step of inducing differentiation of pluripotent stem cells into definitive endoderm cells;
(II) a step of culturing the definitive endoderm cells obtained by the induction of differentiation, in a system containing LY2090314 and/or CHIR99021, followed by induction of differentiation into intestinal progenitor cells;
(III) a step of culturing the intestinal progenitor cells in a system containing LY2090314 and/or a MEK inhibitor to induce differentiation into enterocyte-like cells; and
(IV) a step of dissociating the enterocyte-like cells obtained by the induction of differentiation into single cells, followed by seeding and culturing in a base material for organoid production to produce a cell population of enterocyte-like cells.

11. The method of producing a cell population according to claim 10, wherein a term of the steps (I) to (III) is from 17 days to 27 days after a start of the induction of differentiation of the pluripotent stem cells.

12. The method of producing a cell population according to claim 10, wherein the step of seeding and culturing the enterocyte-like cells in the base material for organoid production in the step (IV) includes a step of culturing the enterocyte-like cells in a medium containing at least Wnt3A, R-spondin, and Noggin.

13. The method of producing a cell population according to claim 10, further comprising the following step (V) after the seeding and culturing of the enterocyte-like cells in the base material for organoid production in the step (IV):
(V) a step of further dissociating the cell population of enterocyte-like cells produced by the seeding in the base material for organoid production into single cells, followed by seeding and culturing in a base material for two-dimensional culture to produce a cell population of enterocyte-like cells.

14. The method of producing a cell population according to claim 13, wherein the step of seeding and culturing the enterocyte-like cells in the base material for two-dimensional culture in the step (V) includes a step of culturing the enterocyte-like cells in a medium containing at least MEM NEAA (product name), B27 supplement (product name), Knockout Serum Replacement (product name), SB431542, PD0325901, LY20903144, and Y-27632.

15. Enterocyte-like cells produced by the method of any one of claims 10 to 14.

16. A medium for inducing differentiation from definitive endoderm cells into intestinal progenitor cells, comprising CHIR99021, retinoic acid, and LY2090314.

FIG. 1

FIG. 2

(1)

(2)

FIG. 3

(1)

|  | day3-4 | day5-6 |
|---|---|---|
| CONDITION 2 | LY | LY |
| CONDITION 3 | LY | CHIR |

(2)

**GENE EXPRESSION LEVEL**

□ CONDITION 2 PD(−)  ▨ CONDITION 2 PD(+)
▥ CONDITION 3 PD(−)  ■ CONDITION 3 PD(+)

(3)

**CYP3A4 activity**

## FIG. 4

(1)

(2)

### GENE EXPRESSION LEVEL

(3)

## CYP3A4 activity

# FIG. 5

**(1)** Human iPS cells

**(2)**

□ day 17 3D  ▤ day 17 2D  ▨ day 27 3D  ▧ day 27 2D  ■ Small intestine

**(3)**

□ day 17 3D  ▤ day 17 2D  ▨ day 27 3D  ▧ day 27 2D  ■ Small intestine

FIG. 6

(1)

### CYP3A4 activity

(2)

### P-gp activity

■ A to B    □ B to A

(3)

### CES2 activity

**FUNCTIONAL ANALYSIS OF ELC MONOLAYERS**

## FIG. 7

(1)

(2)

(3)

(4)

# FIG. 8

FIG. 9

(1)

(2)

## CYP3A4 activity

EGF- : EGF(-)
SB-:SB431542(-)
LY-:LY2090314(-)
VD3-:VitaminD3(-)
PD-:PD0325901(-)
K:   Medium K

(3)

## P-gp activity

FIG. 10

(1)

(2) **CYP3A4 activity**

(3) **TEER**

(4) **P-gp activity**

# FIG. 11

(1)

(2)

(3)

FIG. 12

(1)

Intestinal organoids
(Culture in Medium F)

Formation
of monolayers

Culture in Medium K
for 1 day

Further addition of Medium K to side B
Culture for 2 days

Measurement of Transport Activities
of transporters with RI compound

(2)

P-gp activity

■ A to B    □ B to A

(3)

BCRP activity

■ A to B    □ B to A

FIG. 13

（1）

（2）

（3）

（4）

FIG. 14

(1)

(2)

CYP3A4 activity (P51)

(3)

P-gp activity (P52)

**INTERNATIONAL SEARCH REPORT**

| International application No. |
|---|
| **PCT/JP2023/030444** |

| A. | CLASSIFICATION OF SUBJECT MATTER |
|---|---|

*C12N 5/071*(2010.01)i; *C12N 1/00*(2006.01)i
FI: C12N5/071; C12N1/00 A; C12N1/00 F

According to International Patent Classification (IPC) or to both national classification and IPC

| B. | FIELDS SEARCHED |
|---|---|

Minimum documentation searched (classification system followed by classification symbols)

C12N5/071; C12N1/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | US 2022/0135949 A1 (KOREA RESERCH INSTITUTE OF BIOSCIENCE AND BIOTECHNOLOGY) 05 May 2022 (2022-05-05) claims, examples | 1, 15 |
| Y | claims, examples | 2-14, 16 |
| X | WO 2019/021990 A1 (OSAKA UNIVERSITY) 31 January 2019 (2019-01-31) claims, examples | 6, 7, 10-12, 15 |
| Y | claims, examples | 2-14, 16 |
| X | YAMADA, Shigeru, KANDA, Yasunari, Retinoic acid promotes barrier functions in human iPSC-derived intestinal epithelial monolayers, Journal of Pharmacological Sciences, 2019, vol. 140, pp. 337-344 abstract, results | 15 |
| Y | abstract, results, fig. 1, p. 338, right column, lines 1-4 | 2-14, 16 |
| Y | JP 2019-506861 A (CEDARS-SINAI MEDICAL CENTER) 14 March 2019 (2019-03-14) claims, examples | 2-5, 16 |

☑ Further documents are listed in the continuation of Box C.    ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **01 November 2023** | **14 November 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

International application No.

**PCT/JP2023/030444**

**C.    DOCUMENTS CONSIDERED TO BE RELEVANT**

| Category* | Citation of document, with indication, where appropriate, of the relevant passages | Relevant to claim No. |
|---|---|---|
| X | JP 2022-19411 A (NAGOYA CITY UNIVERSITY) 27 January 2022 (2022-01-27) claims, examples | 15 |
| Y | claims, examples, paragraphs [0059]-[0073] | 6-14 |
| X | JP 2021-122208 A (OSAKA UNIVERSITY) 30 August 2021 (2021-08-30) claims, examples | 15 |
| Y | claims, examples | 13-14 |
| X | WO 2020/091020 A1 (NAGOYA CITY UNIVERSITY) 07 May 2020 (2020-05-07) claims, examples | 15 |
| Y | claims, examples | 13-14 |

Form PCT/ISA/210 (second sheet) (January 2015)

## INTERNATIONAL SEARCH REPORT
### Information on patent family members

International application No.

**PCT/JP2023/030444**

| Patent document cited in search report | | | Publication date (day/month/year) | Patent family member(s) | | | Publication date (day/month/year) |
|---|---|---|---|---|---|---|---|
| US | 2022/0135949 | A1 | 05 May 2022 | WO 2022/098052 A1 claims, examples | | | |
| WO | 2019/021990 | A1 | 31 January 2019 | (Family: none) | | | |
| JP | 2019-506861 | A | 14 March 2019 | WO 2017/136479 A1 claims, examples<br>US 2019/0031992 A1 | | | |
| JP | 2022-19411 | A | 27 January 2022 | (Family: none) | | | |
| JP | 2021-122208 | A | 30 August 2021 | (Family: none) | | | |
| WO | 2020/091020 | A1 | 07 May 2020 | US 2022/0033780 A1 claims, examples | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022143939 A
- WO 2020262492 A1
- WO 2018038042 A1
- JP 2021122208 A
- WO 201820714 A1

**Non-patent literature cited in the description**

- *Nature*, 03 February 2011, vol. 470 (7332), 105-9
- *Stem Cell Reports*, 03 June 2014, vol. 2 (6), 838-52
- *Stem Cells*, June 2013, vol. 31 (6), 1086-96
- *Drug Metab Pharmacokinet*, 2014, vol. 29 (1), 44-51
- *Drug Metab Dispo*, 2015, vol. 43 (4), 603-610
- *Drug Metab Dispo*, October 2016, vol. 44 (10)
- *Nature Microbiology*, March 2019, vol. 4, 492-503
- *Cell*, 2006, vol. 126, 663-676
- *Cell*, 2007, vol. 131, 861-872
- *Natl. Acad. Sci. USA*, 2014, vol. 111 (47), 16772-7
- **M.J. EVANS** ; **M.H. KAUFMAN**. *Nature*, 1981, vol. 292, 154
- *Natl. Acad. Sci. USA*, 1981, vol. 78, 7634